(19)

Europäisches
Patentamt
European
Patent Office
Office européen
des brevets

(11) **EP 4 202 043 A1**

(12) **EUROPEAN PATENT APPLICATION**

(43) Date of publication:
**28.06.2023 Bulletin 2023/26**

(21) Application number: **21217144.1**

(22) Date of filing: **22.12.2021**

(51) International Patent Classification (IPC):
**C12N 9/22** *(2006.01)*       **C12P 7/64** *(2022.01)*
**C12N 15/52** *(2006.01)*       **C12N 15/90** *(2006.01)*
**C12N 15/11** *(2006.01)*

(52) Cooperative Patent Classification (CPC):
**C12N 9/22; C12N 1/00; C12N 15/113; C12N 15/52; C12N 15/905; C12P 7/64;** C12N 2310/20; C12R 2001/645

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Technische Universität München 80333 München (DE)**

(72) Inventors:
• **Shaigani, Pariya**
  **82140 Olching (DE)**
• **Brück, Thomas**
  **85452 Eichenried (DE)**
• **Mehlmer, Norbert**
  **85748 Garching bei München (DE)**

(74) Representative: **Engelhard, Markus Boehmert & Boehmert Anwaltspartnerschaft mbB Pettenkoferstrasse 22 80336 München (DE)**

Remarks:
The complete document including Reference Table(s) and the Sequence Listing(s) can be downloaded from the EPO website

(54) **A METHOD FOR GENETIC MODIFICATION FOR HIGH CG CONTENT MICROORGANISMS**

(57)    The present invention relates to a method of genetically modifying a GC rich microorganism. The present invention further relates to a genetically modified GC rich microorganism. Furthermore, the present invention relates to a composition comprising a RNA-guided endonuclease, at least one guide RNA (gRNA), and optionally donor DNA. The present invention also relates to a method of preparing a target compound, e.g. an acetyl-CoA-based hydrophobic compound, and/or an oil having a specific fatty acid profile, e.g. high oleic oil, using a genetically modified GC rich microorganism.

**EP 4 202 043 A1**

**Description**

FIELD OF THE INVENTION

The present invention relates to a method of genetically modifying a GC rich microorganism. The present invention further relates to a genetically modified GC rich microorganism. Furthermore, the present invention relates to a composition comprising a RNA-guided endonuclease, at least one guide RNA (gRNA), and optionally donor DNA. The present invention also relates to a method of preparing a target compound, e.g. an acetyl-CoA-based hydrophobic compound, and/or an oil having a specific fatty acid profile, e.g. high oleic oil, using a genetically modified GC rich microorganism.

BACKGROUND OF THE INVENTION

Increasing the global demand for plant- and animal-based lipids in biofuel, pharmaceutical and oleochemical industries have had negative impacts on the biodiversity and resulted in land-use changes. Consequently, microbial oils have attracted attention as alternative to vegetable oils. Oleaginous microorganisms, such as the oleaginous yeast *Cutaneotrichosporon oleaginosus* ATCC 20509, can accumulate lipids through the "de novo" lipid biosynthesis pathway. Oleaginous microorganisms such as *C. oleaginosus* grow on cost efficient, undetoxified and complex waste biomass streams, and can be used to produce microbial lipids.

Genetic engineering is a route for improving and diversifying compounds produced by a microorganism, e.g. high value compounds such as triglycerides, terpenoids and other metabolically derived value adding compounds. The modification and optimization of high value products, for instance tailor made lipids, generated by oleaginous yeasts have been reported [1]. However, their productivity and product diversity is restricted by lack of efficient genetic tools.

To date, Agrobacterium mediated transformation (AMT) has been established for stable random integration of expression cassettes into the genome of GC rich microorganisms, particularly oleaginous microorganisms such as *C. oleaginosus* [1]. Such random integration of genes in the genome results in different and unpredictable gene expression levels, which mainly depends on variable insertion loci and the number of insertion events. The dominance of non-homologous end joining (NHEJ) to homologous recombination (HDR) in GC rich microorganisms, particularly oleaginous microorganisms such as *C. oleaginosus,* as well as a lack of suitable plasmids for GC rich microorganisms, e.g. a lack of an artificial plasmid for oleaginous yeasts, have impeded the targeted and extensive genetic engineering [2]. To that end, the establishment of an efficient genomic editing tool for targeted gene modification is needed to enable more precise and advanced engineering of GC rich microorganisms, particularly oleaginous microorganisms having a high GC content e.g. *C. oleaginosus.*

The dominance of non-homologous end joining (NHEJ) to homologous recombination (HDR) has been an obstacle in the genetic modification of GC rich microorganisms such as *C. oleaginosus.* Particularly, genetic engineering of GC rich microorganisms, such as oleaginous microorganisms having a high GC content, e.g. a GC content of ≥ 50 or 60%, and having a high amount of repetitive sequences, is very difficult due to the presence of such repetitive sequences and the high GC content resulting in off-target effects and nucleic acid secondary structure formation.

The reported protocols for genetic modifications of GC rich microorganisms, such as oleaginous microorganisms, so far have only described random gene insertion in the genome and random mutagenesis. Thus, there is a need for methods of genetically modifying GC rich microorganisms, such as oleaginous microorganisms, in a targeted way. Furthermore, there is a need for methods of genetically modifying GC rich microorganisms, particularly oleaginous microorganisms having a high GC content, such as *C. oleaginosus.* There is also the need for means of genetically modifying such GC rich microorganisms in a targeted way, e.g. for increasing the production of target compounds such as acetyl-CoA-based hydrophobic compounds and/or certain fatty acids.

SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a method of genetically modifying a GC rich microorganism, optionally for increasing the production of acetyl-CoA-based hydrophobic compounds in said microorganism, comprising the following steps:

i) providing a GC rich microorganism, preferably an oleaginous microorganism, more preferably an oleaginous yeast;
ii) optionally, pretreating said GC rich microorganism; wherein, preferably, said pretreating comprises spheroplasting said GC rich microorganism;

iii) transforming said GC rich microorganism with a RNA-guided endonuclease, at least one guide RNA, and optionally a donor DNA;

iv) optionally, selecting a transformed cell of said GC rich microorganism;

v) obtaining a genetically modified GC rich microorganism.

In one embodiment, said GC rich microorganism has a guanine-cytosine (GC) content of at least 50%, preferably at least 55%, more preferably at least 58%, even more preferably at least 60%.

In one embodiment, said GC rich microorganism is selected from yeasts, fungi, bacteria and microalgae; wherein, preferably, said GC rich microorganism is an oleaginous microorganism; wherein, more preferably, said GC rich microorganism is an oleaginous yeast; wherein, even more preferably, said GC rich microorganism is selected from *Rhodosporidium sp., Yarrowia sp., Rhodotorula sp., Candida sp., Lipomyces sp., Cutaneotrichosporon sp., Trichosporon sp.,* preferably selected from *Cutaneotrichosporon sp.,* more preferably *Cutaneotrichosporon oleaginosus* (ATCC 20509); and/or said GC rich microorganism has a guanine-cytosine (GC) content of at least 50%, preferably at least 55%, more preferably at least 58%, even more preferably at least 60%.

In one embodiment, said RNA-guided endonuclease is a CRISPR-associated (Cas) endonuclease, preferably selected from Cas9, Cas1, Cas2, Cas4, Cas3, Cas10, Casi2, Cas13, Csm, scfi, or variants thereof, e.g. Casi2a, dCas9, D10A CAS9 nickase or H840A CAS9 nickase.

In one embodiment, said transforming in step iii) comprises applying said RNA-guided endonuclease in the form of a RNA-guided endonuclease protein, in the form of a DNA encoding said RNA-guided endonuclease, or in the form of a mRNA encoding said RNA-guided endonuclease to said GC rich microorganism, preferably in the form of a mRNA encoding said RNA-guided endonuclease.

In one embodiment, said at least one guide RNA comprises CRISPR RNA (crRNA), trans-activating CRISPR RNA (tracrRNA), and/or single-guide RNA (sgRNA); wherein, preferably, said at least one guide RNA comprises a first guide RNA and a second guide RNA, wherein a sequence of said first guide RNA is different from a sequence of said second guide RNA.

In one embodiment, said donor DNA comprises a DNA repair template, a DNA encoding a selection marker, and/or a gene or sequence of interest, e.g. a gene involved in the production of a target compound such as a fatty acid.

In one embodiment, said transforming in step iii) comprises applying said RNA-guided endonuclease and said at least one guide RNA, preferably jointly, in the form of a ribonucleoprotein complex.

In one embodiment, said transforming in step iii) comprises applying said RNA-guided endonuclease in the form of a mRNA encoding said RNA-guided endonuclease, and wherein said at least one guide RNA comprises sgRNA.

In one embodiment, said pretreating in step ii) comprises an enzymatic pretreatment, chemical pretreatment, and/or another spheroplasting procedure; wherein, preferably, said pretreating in step ii) comprises an enzymatic pretreatment performed using at least one enzyme selected from glycosyl hydrolases, cellulase, hemicellulase, mannanase, xyloglucanse, xylanase, glucanase, glucosidase, arabinase, amylase, fructanase, laminarase, hydrolase, protease, or any combination thereof, preferably a combination of cellulase, hemicellulase, mannanase, xyloglucanse, xylanase, glucanase, and glucosidase.

In one embodiment, said pretreating in step ii) comprises a treatment of said microorganism with a hydrolase alone, or a hydrolase in combination with/followed by a protease;

wherein, optionally, said hydrolase is selected from hydrolases produced by a fungus, preferably a filamentous fungus, more preferably a fungus selected from the *Trichoderma sp., Aspergillus sp., Penicillium sp., Aureobasillium sp.* and *Fusarium sp.,* even more preferably *Trichoderma reesei;* and/or wherein, optionally, said protease is selected from proteases produced by *Aspergillus sp., Streptomyces sp.* or *Bacillus sp.*

In one embodiment, said transforming in step iii) is performed using electroporation; PEG-based or other nanoscale carrier-based transformation; biological ballistics; glass bead transformation; vesicle-mediated delivery; a viral transfection system, e.g. lentivirus (LVs), adenovirus (AdV), or adeno-associated virus (AAV); liposomal delivery, e.g. lipofection; a chemical transfection technique; or combinations thereof; preferably using electroporation and/or PEG-based transformation; more preferably using electroporation.

In one embodiment, said method comprises said selecting in step iv), wherein said selecting in step iv) comprises subjecting said microorganism to a selective agent selective for a selection marker, optionally a selection marker encoded by said donor DNA;

wherein, preferably, said selecting comprises subjecting said microorganism to a selective agent at a concentration in the range of from 10 % to 90 %, preferably 10 % to 70 %, more preferably 10 % to 60 %, of the minimum selectable concentration; and/or subjecting said microorganism to a selective agent at a concentration in the range of from 90 % to 100 %, preferably 99 % to 100 %, of the minimum selectable concentration; wherein, more preferably, said selecting comprises subjecting said microorganism to said selective agent at a concentration in the range of from 10 % to 90 %, preferably 10 % to 70 %, more preferably 10 % to 60 %, of the minimum selectable concentration in a first medium, e.g. a liquid medium or solid medium, preferably in an upper layer of agar, and, optionally subsequently, subjecting said microorganism to a selective agent at a concentration in the range of from 90 % to 100 %, preferably 99 % to 100 % in a second medium, e.g. a liquid medium or solid medium, preferably in a lower layer of agar.

In a further aspect, the present invention relates to a genetically modified GC rich microorganism, preferably an oleaginous microorganism, more preferably an oleaginous yeast, even more preferably *Cutaneotrichosporon oleaginosus,* comprising a RNA-guided endonuclease, at least one guide RNA, and optionally a donor DNA; wherein, optionally, said RNA-guided endonuclease and said at least one guide RNA are present in said cell in the form of a ribonucleoprotein complex.

In one embodiment, the genetically modified GC rich microorganism is obtained using a method as defined herein.

In this aspect, the GC rich microorganism, the oleaginous microorganism, the oleaginous yeast, the RNA guided endonuclease, the at least one guide RNA, the donor DNA, and the ribonucleoprotein complex are as defined herein.

In a further aspect, the present invention relates to a composition comprising

- a RNA-guided endonuclease; optionally a RNA-guided endonuclease protein, a DNA encoding said RNA-guided endonuclease, or a mRNA encoding the RNA-guided endonuclease, preferably a mRNA encoding the RNA-guided endonuclease;
  wherein, preferably, said RNA-guided endonuclease is a CRISPR-associated (Cas) endonuclease, more preferably selected from Cas9, Cas1, Cas2, Cas4, Cas3, Cas10, Casi2, Cas13, Csm, scfi, or variants thereof, e.g. Casi2a, dCas9, D10A CAS9 nickase or H840A CAS9 nickase, even more preferably Cas9 endonuclease such as Cas9 endonuclease having a sequence of any of SEQ ID NO. 1-4;
- at least one guide RNA (gRNA); preferably crRNA, tracrRNA, and/or sgRNA;
  wherein, optionally, said gRNA comprises a tracrRNA sequence of SEQ ID NO. 5 and/or comprises a crRNA sequence of any one of SEQ ID NOs. 14-26;
- optionally, donor DNA; preferably comprising a DNA repair template, a DNA encoding a selection marker, and/or a gene or sequence of interest, e.g. a gene involved in the production of a target compound such as a fatty acid;

    wherein, optionally, said selection marker is Ura5;

wherein, optionally, said gene or sequence of interest encodes delta-9-desaturase, delta-12-desaturase, elongase, oleat hydratase, aldo keto reductase promoter, aldo keto reductase terminator, transcription elongation factor 2 promoter, TEF promoter, and/or TEF terminator;
wherein, optionally, said donor DNA comprises a sequence of any of SEQ ID NO. 6-13 and 27.

In this aspect, the RNA guided endonuclease, the at least one guide RNA, and the donor DNA, are as defined herein.

In a further aspect, the present invention relates to a plasmid or plasmid collection comprising

- a mRNA encoding a RNA-guided endonuclease; wherein, preferably, said RNA-guided endonuclease is Cas9 endonuclease, more preferably Cas9 endonuclease having a sequence of any of SEQ ID NO. NO. 1-4;
- at least one guide RNA (gRNA); preferably crRNA, tracrRNA, and/or sgRNA;
  wherein, optionally, said gRNA comprises a tracrRNA sequence of SEQ ID NO. 5 and/or comprises a crRNA sequence of any one of SEQ ID NOs. 14-26;
- optionally, donor DNA, e.g. a DNA repair template; wherein, optionally, said donor DNA has a sequence of any of SEQ ID NO. 6-13 and 27.

In this aspect, the RNA guided endonuclease, the at least one guide RNA, and the donor DNA, are as defined herein.

In a further aspect, the present invention relates to a method of preparing a target compound, e.g. acetyl-CoA or an acetyl-CoA-based hydrophobic compound, and/or an oil having a specific fatty acid profile, e.g. high oleic oil, using a genetically modified GC rich microorganism, preferably oleaginous microorganism, more preferably oleaginous yeast, wherein said method comprises:

a) providing a genetically modified GC rich microorganism, preferably oleaginous microorganism, more preferably oleaginous yeast, using a method as defined above; wherein said method comprises transforming said microorganism with a donor DNA, wherein said donor DNA comprises a gene or sequence of interest, e.g. a gene involved in the production of said target compound and/or specific fatty acid;
b) growing said genetically modified GC rich microorganism;
c) obtaining said target compound and/or oil having a specific fatty acid profile;

wherein, preferably, said target compound is selected from saturated short-chain fatty acids, saturated medium-chain fatty acids, saturated long-chain fatty acids, monounsaturated fatty acids, polyunsaturated fatty acids, functionalized fatty acids, ergosterol, ergosterol derivatives, terpenes, alkaloids, acetyl-CoA-based synthetic compounds, tocochromanols, e.g. α-tocopherol and α-tocotrienol, monoterpenoids, sesquiterpenoids, diterpenoids, squalene, carotenoids, triterpenes, pheophytins, vitamins, citric acid, volatile fatty acids, oxalic acid, lactic acid, malic acid, and exopolysaccharides.

In this aspect, the GC rich microorganism, the oleaginous microorganism, the oleaginous yeast, and the donor DNA are as defined herein.

DETAILED DESCRIPTION

The dominance of non-homologous end joining (NHEJ) to homologous recombination (HDR) in GC rich microorganisms, e.g. oleaginous microorganisms such as *C. oleaginosus,* has impeded the targeted and extensive genetic engineering of oleaginous microorganisms. Furthermore, genetic engineering of oleaginous microorganisms has been hampered by a lack of artificial plasmids for GC rich microorganisms, such as oleaginous microorganisms. Furthermore, delivery of genes and/or endonucleases inside cell wall containing GC rich microorganisms, e.g. oleaginous microorganisms, has been an obstacle due to the presence of a cell wall.

It is an aim of the invention to provide an enhanced transformation system, e.g. a method for genetic modification of GC rich microorganisms in a targeted way. Particularly, it is an aim of the invention to provide enhanced means for genetically modifying GC rich microorganisms. Furthermore, it is an aim of the invention to provide genetically modified microorganisms capable of efficiently producing target compounds, such as acetyl-CoA-based hydrophobic compounds, and/or to provide a genetically modified microorganism having a modified acetyl-CoA pool. It is also an aim of the invention to efficiently produce target compounds, such as acetyl-CoA based hydrophobic compounds, using GC rich microorganisms such as oleaginous microorganisms. A further aim of the invention is to efficiently transform GC rich microorganisms, such as genetically modifying the GC rich microorganisms to produce target compounds, e.g. acetyl-CoA-based hydrophobic compounds. It is also an aim of the invention to provide a method to genetically modify GC rich microorganisms at defined locations of the genome. Furthermore, the invention aims at enhancing efficiency of transformation of a GC rich microorganism.

The method of genetically modifying a GC rich microorganism according to the invention is highly advantageous in that the cell wall is degraded prior to transformation. For example, an enzymatic pretreatment is used to degrade the cell wall of a GC rich microorganism, preferably an oleaginous microorganism. By degrading the cell wall of the GC rich microorganism, gene transfer, RNA transfer, protein transfer and/or transfer of a ribonucleoprotein complex is facilitated. Advantageously, the enzymatic pretreatment and/or spheroplasting procedure can be specifically adapted to the respective microorganism to be treated, e.g. the enzymes to be used can be adapted to the components of the cell wall of the respective microorganism. Thus, advantageously, a method of the invention allows to provide a tailored enzyme system for pretreating the GC rich microorganism.

Furthermore, the method of genetically modifying according to the invention advantageously allows to transfer even ribonucleoprotein complexes to GC rich microorganisms. The inventors have successfully developed an efficient Cas-mediated genome editing technique through employment of CAS mRNA and ribonucleoprotein (RNP). An advantage of using the RNA-guided endonuclease in the form of mRNA or in the form of a protein, particularly a ribonucleoprotein, is that promoter efficiencies and expression levels of the RNA-guided endonuclease genes e.g. CAS genes do not have to be taken into account. Thus, advantageously, the method of genetically modifying according to the present invention can be performed in the form of a DNA-free genome editing technique. An advantage of RNA-guided endonuclease in the form of mRNA or in the form of a RNP is that RNPs are degraded over time, and the Cas mRNA results in a transient expression of CAS protein, which results in considerably less cloning effort and alleviated off-target effects. The method of genetically modifying according to the invention is highly advantageous in that even microorganisms with high GC content can be genetically modified.

To even further increase the specificity of the genetic modification, multiple sgRNA molecules can be used to transform the GC rich microorganism. For example, sgRNA molecules targeting the same or different target sequences can be used to increase the specificity. In one embodiment, the method of genetically modifying a GC rich microorganism comprises transforming said microorganism with one or more sgRNA molecules, preferably multiple sgRNA molecules targeting different target sequences. In one embodiment, the method of genetically modifying a GC rich microorganism comprises transforming said microorganism with one or more tracrRNA and one or more crRNA molecules, preferably multiple crRNA molecules targeting different target sequences.

The genome of GC rich microorganisms, e.g. oleaginous microorganisms such as *C. oleaginous,* is rich in GC content (e.g. at about 61%), which complicates the design of guide RNA e.g. sgRNA design. While the 20 bp sgRNA is believed to specify an RNA guided endonuclease such as CAS9 to a targeted sequence, it has been shown that three to five mismatches in the PAM-distal part can be tolerated. In addition to the PAM, the seed sequence of sgRNA, which is 10 to 12 bp directly adjacent to the PAM, is generally playing a role in on-target binding of CAS9:sgRNA complex. Therefore, the existence of similar and repetitive sequences in the genome of GC rich microorganisms, e.g. oleaginous microorganisms having a high GC content such as *C. oleaginosus,* contributes to the off-target effects of CAS9:sgRNA. Derivatives of CAS9 nickases (D10A, H840A) were created by mutating one of two Cas9 nuclease domains, resulting in a single-strand break in the DNA. Hence, these CAS9 derivatives are provided with two adjacent gRNAs targeting opposite DNA strands, to generate the required double-strand break. Hence, they decrease the off-target effects and facilitate the gRNA design.

The previously established methods cannot be used for direct delivery of the CAS protein/mRNA, synthetic sgRNAs and donor DNAs to oleaginous microorganisms having a high GC content such as *C. oleaginosus.* Therefore, a more flexible transformation approach for GC rich microorganisms, e.g. oleaginous yeasts and specifically *C. oleaginosus,* was established, which enables an efficient RNA-guided endonuclease delivery, preferably Cas delivery. Electroporation of spheroplasts e.g. *C. oleaginosus* spheroplasts provides a less laborious and faster procedure for delivery of all types of gene elements, for instance ssDNA/dsDNA fragments, DNAs, mRNA, small RNAs as well as proteins, such as nucleases. An optimized and efficient spheroplast isolation was achieved by treating the GC rich microorganisms e.g. oleaginous microorganisms, preferably oleaginous yeast cells, with an enzyme mixture isolated from an enzyme-producing organism, preferably a filamentous fungus, even more preferably *Trichoderma reesei.* For example, a hydrolase enzyme system used for spheroplast preparation was generated by fermenting *T. reesei* on *C. oleaginous* biomass cultivated under non-limiting conditions. Furthermore, the hydrolase enzyme system for spheroplasting was generated by cultivation of *T. reesei* on C.o. biomass, that was generated under oil forming cultivation conditions. Moreover, in an exemplary hydrolase production for downstream spheroplasting such as *C. oleaginosus* spheroplasting, the oil containing oleaginous microorganism biomass was subjected either sequentially or combined to an organic phase (i.e. methanol) and water wash prior to fermentation with *T. reesei.*

In one embodiment, an enzyme-producing microorganism such as a fungus, preferably filamentous fungus, produces the at least one enzyme used for said pretreating in step ii). In one embodiment, said at least one enzyme used for pretreating in step ii) is obtained from an enzyme-producing microorganism such as a fungus, preferably filamentous fungus, e.g. any enzyme selected from glycosyl hydrolases, cellulase, hemicellulase, mannanase, xyloglucanse, xylanase, glucanase, glucosidase, arabinase, amylase, fructanase, laminarase, hydrolase, protease, or any combination thereof, is obtained from a fungus, preferably filamentous fungus. In one embodiment, said enzyme is produced by said enzyme-producing microorganism such as a fungus, preferably filamentous fungus, and is subsequently obtained from a fermentation broth of said fungus by salting out said enzyme, by chromatography, and/or filtration, such as nanofiltration and/or ultrafiltration, said obtaining optionally further comprising a drying procedure e.g. spray drying. In one embodiment, the pretreating in step ii), preferably spheroplasting, is performed using a tailored enzyme system, wherein the tailored enzyme system is said at least one enzyme produced by an enzyme-producing filamentous fungus cultivated with an inducing system, wherein the inducing system comprises or consists of said GC rich microorganism or a component thereof.

The inventors have developed a gene editing system using an RNA-guided endonuclease, particularly CRISPR/CAS system, for GC rich microorganisms, e.g. oleaginous microorganisms having a high GC content such as *C. oleaginosus,* for targeted engineering of such GC rich microorganisms. The method of the invention is advantageous as off-target effects are reduced. Off-target effects were reduced by sequence alignments between the crRNA sequences and the genomic DNA of the GC rich microorganisms e.g. *C. oleaginosus.* Particularly, the off targets were identified by sequence alignments between the crRNA sequences and the genomic DNA, then the crRNA sequences without unspecific targets were selected. A library of crRNA sequences was built, afterwards the library was screened by sequence alignment to the genomic DNA and those without unspecific targeting were selected. RNA-guided endonucleases such as a CAS:sgRNA RNP complex, as well as its variants such as D10A together with two sgRNAs, were successfully delivered to GC rich microorganisms such as *C. oleaginosus.* Moreover, targeted gene editing was successfully accomplished via transformation of spheroplasts e.g. yeast spheroplasts with a RNA-guided endonuclease in the form of mRNA (e.g. Cas mRNA) and sgRNA. Furthermore, new sequences were introduced through simultaneous transfer of single stranded or double stranded donor DNAs resulting in ura5 knockout mutants. The mutants were selected on YNB-5foa plates containing uracil. Further analysis was done by gene sequencing. In one embodiment, the guide RNA, e.g. crRNA, is selected such that no site other than the target site is detected on the genomic DNA. In one embodiment, by selecting the guide RNA, e.g. crRNA, such that no site other than the target site is detected on the genomic DNA, off-target activities of the endonuclease are reduced. In one embodiment, off-target effects are further reduced by using a nickase. In one embodiment, delivery of endonuclease protein or endonuclease encoding mRNA decreases off-target effects compared to delivery of a gene encoding the nuclease which is incorporated into the genome and stays active permanently. In a preferred embodiment, two sgRNAs are delivered to the GC rich microorganism, e.g. in combination with a endonuclease variant such as D10A CAS9 nickase or H840A CAS9 nickase. Such combination of an endonuclease with two sgRNAs even further increases the efficiency of the method.

The method of the invention using a RNA-guided endonuclease, e.g. CRISPR/CAS or its variants such as D10A and H840A, provides a precise, flexible, and straightforward approach for targeted engineering of GC rich microorganism, particularly oleaginous yeasts such as *C. oleaginosus*. The method of the invention allows to fully employ these important microorganisms, such as *C. oleaginosus,* as an industrial cell factory, and to optimize the metabolically derived products such as acetyl-CoA or acetyl-CoA based hydrophobic compounds for a broader range of applications. For example, the method of the invention can be used to prepare microbial oil with customized fatty acid profile, medically active compounds, such as ergosterol, ergosterol derivatives, tocochromanols (such as α-tocopherol, and α-tocotrienol), mono-, sesqui- and diterpeneoids, squalene, carotenoids, triterpenes, pheophytins, and vitamins. Furthermore, the method of the invention allows to prepare a genetically modified GC rich microorganism with high tolerance to toxic compounds and/or with the ability to effectively utilize a wide spectrum of complex feedstocks and waste streams.

In one embodiment, the term "genetically modifying", as used herein, relates to modifying the genetic information of an organism, preferably a GC rich microorganism such as an oleaginous microorganism. For example, such genetically modifying may comprise knock in of a gene, knock out of a gene, and/or point mutation. By genetically modifying a GC rich microorganism, such as oleaginous microorganism, features of interest can be modified, e.g. the production of acetyl-CoA-based hydrophobic compounds can be increased and/or a specific fatty acid profile of the microbial lipids produced by said GC rich microorganism can be achieved. In one embodiment, the method of genetically modifying according to the present invention is advantageous since a GC rich microorganism, preferably an oleaginous microorganism having a high GC content, can be modified to have a feature of interest, such as an increased production of acetyl-CoA-based hydrophobic compounds and/or a production of a specific fatty acid profile. In one embodiment, the method of genetically modifying a GC rich microorganism of the invention is a method for increasing the production of acetyl-CoA-based hydrophobic compounds in said microorganism and/or for modification or modulation of the acetyl-CoA pool of said microorganism. For example, by modifying one or more genes involved in acetyl-CoA synthesis, the acetyl-CoA level can be modified, which is a precursor for many acetyl-CoA-derived products such as acetyl-CoA-based hydrophobic compounds. Moreover, by modifying one or more genes involved in the metabolism of acetyl-CoA-based compounds, these compounds can also be modulated and modified.

In one embodiment, a method of genetically modifying GC rich microorganisms allows for gene modification, e.g. increased expression or decreased expression of the modified gene, gene deletion, gene insertion, frame shift, point mutation, and/or gene substitution.

The term "GC rich microorganism", as used herein, relates to a microorganism having a guanine-cytosine (GC) content of at least 50%, preferably at least 55%, more preferably at least 58%, even more preferably at least 60%, e.g. about 61%. GC content (or guanine-cytosine content) is the percentage of nitrogenous bases in a DNA or RNA molecule, e.g. a genome, that are either guanine (G) or cytosine (C). In one embodiment, the GC content indicates the proportion of G and C bases out of all nucleic acid bases, also including adenine and thymine in DNA and adenine and uracil in RNA. In one embodiment, the term "GC content", as used herein, relates to the GC content of the genome of the GC rich microorganism and/or to the GC content of all nucleic acids comprised by said GC rich microorganism. In one embodiment, the GC content is calculated as

$$\frac{G+C}{A+T+G+C} \times 100\%$$

. In one embodiment, the GC content is measured by sequencing the genome and/or the total nucleic acid content of the microorganism. In one embodiment, the GC rich microorganism comprises a cell wall. In a preferred embodiment, the GC rich microorganism is an oleaginous microorganism, e.g. an oleaginous yeasts. In one embodiment, a GC rich microorganism is a microorganism having a genome with a GC content of at least 50%, preferably at least 55%, more preferably at least 58%, even more preferably at least 60%, e.g. about 61%. In one embodiment, the GC rich microorganism is an oleaginous microorganism having a genome with a GC content of at least 50%, preferably at least 55%, more preferably at least 58%, even more preferably at least 60%, e.g. about 61%. In a preferred embodiment, the GC rich microorganism is an oleaginous microorganism, i.e. a GC rich oleaginous microorganism, for example an oleaginous yeast such as *Cutaneotrichosporon oleaginosus*. Oleaginous microorganisms are known to a person skilled in the art. For example, oleaginous microorganisms such as oleaginous yeasts, oleaginous fungi, oleaginous bacteria, and oleaginous microalgae are microorganisms typically capable of producing microbial lipids, e.g. microorganisms that accumulate more than 20% w/w of lipids on a cell dry weight basis. In one embodiment, a GC rich microorganism is selected from oleaginous microorganisms, preferably selected from oleaginous yeasts, oleaginous fungi, oleaginous bacteria and oleaginous microalgae. In one embodiment, said oleaginous yeasts are selected from *Rhodosporidium sp., Yarrowia sp., Rhodotorula sp., Candida sp., Lipomyces sp., Cutaneotrichosporon sp., Trichosporon sp.,* preferably selected from *Cutaneotrichosporon sp.,* more preferably is *Cutaneotrichosporon oleaginosus.* If said oleaginous microorganism is a yeast, it is an oleaginous yeast, wherein preferably, said oleaginous yeast is selected from *Rhodosporidium sp., Yarrowia sp., Rhodotorula sp., Candida sp., Lipomyces sp., Cutaneotrichosporon sp., Trichosporon sp.,* preferably selected from *Cutaneotrichosporon sp.,* more preferably *Cutaneotrichosporon oleaginosus.* If said oleaginous microorganism is a fungus, it is an oleaginous fungus, wherein preferably, said oleaginous fungus is selected from the genus *Cunninghamella, Aspergillus, Mortierella* and *Humicola.* If said oleaginous microorganism is a bacterium, it is an oleaginous bacterium, wherein, preferably, said oleaginous bacterium is selected from the genus *Rhodococcus, Acinetobacter* and *Bacillus.* Preferred species of oleaginous bacteria are *Rhodococcus opacus, Acinetobacter calcoaceticus* and *Bacillus alcalophilus.* If said oleaginous microorganism is a microalga, it is an oleaginous microalga, wherein, preferably, said microalga is selected from the genus *Chlorella, Pseudochlorococcum, Nannochloris, Nannochloropsis, Isochrysis, Tribonema* such as *Tribonema minus, Dunaliella, Ankistrodesmus, Botryococcus* such as *Botryococcus braunii, Pavlova, Scenedesmus, Skeletonema* and *Nitzschia.*

In one embodiment, said GC rich microorganism is an oleaginous yeast selected from *Rhodosporidium sp., Yarrowia sp., Rhodotorula sp., Candida sp., Lipomyces sp., Cutaneotrichosporon sp., Trichosporon sp.,* preferably selected from *Cutaneotrichosporon sp.,* more preferably *Cutaneotrichosporon oleaginosus.* A particularly preferred species of oleaginous yeast is *Cutaneotrichosporon oleaginosus.* In one embodiment, said *Cutaneotrichosporon oleaginosus* is *Cutaneotrichosporon oleaginosus* ATCC 20509. In one embodiment, said oleaginous microorganism has a guanine-cytosine (GC) content of at least 50%, preferably at least 55%, more preferably at least 58%, even more preferably at least 60%, e.g. about 61%.

The term "acetyl-CoA-based hydrophobic compound", as used herein, relates to a hydrophobic compound which comprises acetyl-CoA, and/or which is produced involving acetyl-CoA and/or involving an acetyl-CoA metabolism. In one embodiment, an acetyl-CoA-based compound, preferably acetyl-CoA-based hydrophobic compound, is any compound selected from saturated short-chain fatty acids, saturated medium-chain fatty acids, saturated long-chain fatty acids, monounsaturated fatty acids, polyunsaturated fatty acids, functionalized fatty acids, ergosterol, ergosterol derivatives, terpenes, alkaloids, acetyl-CoA-based synthetic compounds, tocochromanols, e.g. α-tocopherol and α-tocotrienol, monoterpenoids, sesquiterpenoids, diterpenoids, squalene, carotenoids, triterpenes, pheophytins, vitamins, citric acid, volatile fatty acids, oxalic acid, lactic acid, malic acid, and exopolysaccharides. In one embodiment, the term "short-chain fatty acid" relates to a fatty acid having 5 or less carbon atoms. In one embodiment, the term "medium-chain fatty acid" relates to a fatty acid having 6 to 12 carbon atoms. In one embodiment, the term "long-chain fatty acid" relates to a fatty acid having 12 or more carbon atoms. In one embodiment, a target compound is an acetyl-CoA-based hydrophobic compound and/or fatty acid.

In one embodiment, the term "spheroplasting", as used herein, relates to partially or fully removing the cell wall of a microbial cell, preferably of a GC rich microorganism such as an oleaginous microorganism. For example, a spheroplast is a microbial cell partially devoid or fully devoid of the cell wall. In one embodiment, by spheroplasting the GC rich microorganism, sugars, lipids, and proteins of the cell wall of the GC rich microorganism are removed. In one embodiment, by removing and/or lysing the cell wall of a GC rich microorganism e.g. oleaginous microorganism, the microorganism is made amenable for transfection. Typically, when the microbe's cell wall is digested to obtain a spheroplast, membrane tension causes the cell to acquire a spherical shape. Advantageously, by spheroplasting said GC rich microorganism e.g. oleaginous microorganism, the GC rich microorganism becomes amenable for transfection. The inventors have surprisingly found that, after pretreating a GC rich microorganism such as an oleaginous microorganism, thereby providing a spheroplast of said GC rich microorganism, said GC rich microorganism can be efficiently transfected, such as transfected using an RNA-guided endonuclease, e.g. in the form of an mRNA. In one embodiment, the GC rich microorganism is a microorganism comprising a cell wall. In one embodiment, the GC rich microorganism has a cell wall. In one embodiment, if the GC rich microorganism comprises a cell wall, the method of genetically modifying a GC rich microorganism comprises pretreating said microorganism, preferably comprises spheroplasting said microorganism.

In one embodiment, the term "pretreating", as used herein, relates to treating said GC rich microorganism, preferably oleaginous microorganism, prior to a transfection of said GC rich microorganism, preferably to increase the susceptibility of said GC rich microorganism to transfection. In one embodiment, by performing such pretreatment of step ii), the transfection efficiency of a transfection of such pretreated GC rich microorganism, e.g. such pretreated oleaginous microorganism, is increased compared to a transfection of a GC rich microorganism without such pretreatment. In one embodiment, said pretreating in step ii) comprises an enzymatic pretreatment, chemical pretreatment, and/or another spheroplasting procedure. In one embodiment, said pretreating is an enzymatic pretreating comprising contacting said GC rich microorganism, preferably oleaginous microorganism, with said at least one enzyme. Preferably, such at least one enzyme, e.g. enzyme mix, is obtained from another microorganism selected from filamentous fungi and bacteria, preferably a filamentous fungus. In one embodiment, the fungus is selected from the Genus *Trichoderma, Aspergillus, Penicillium, Aureobasillium* and *Fusarium.* In a more preferred embodiment, the filamentous fungus is *Trichoderma reesei,* because this has proved to produce a particularly efficient enzyme mix that allows a pretreatment of the cell wall of the GC rich microorganism e.g. oleaginous microorganism. In one embodiment, said at least one enzyme is obtained from a fungus, preferably a filamentous fungus that has been cultured in the presence of an inducing system, wherein, preferably, said inducing system is a component of said GC rich microorganism, preferably one or several cell-wall components of said GC rich microorganism, so as to obtain an enzyme preparation that allows a pretreatment of said GC rich microorganism's cell wall to improve the cell wall's permeability for transfection. Preferably, such enzyme preparation is produced by culturing said filamentous fungus in the presence of cell wall fragments of said GC rich microorganism. Without wishing to be bound by any theory, the present inventors believe that exposure of the filamentous fungus, e. g. *T. reesei,* to the presence of such cell wall component of said GC rich microorganism allows such filamentous fungus to produce exactly the suitable enzyme(s) to complete the lysis of the cell-walls of the GC rich microorganism. In a particularly preferred embodiment, a filamentous fungus from the genus *Trichoderma* is used, e. g. *Trichoderma reesei,* and in a particularly preferred embodiment, a mutant of *Trichoderma reesei* is used, such as the mutant having the ATCC deposition number(s) 56765 and 13631. Once the filamentous fungus has been cultivated, the resultant culture may be further processed, such as concentrated, and the biomass of the fungus itself is removed, and the resultant supernatant may be used in such a form, or it may be freeze-dried and kept for storage and subsequent reconstitution in an appropriate aqueous solution.

In one embodiment, said pretreating in step ii) comprises an enzymatic pretreatment performed using at least one enzyme, preferably selected from glycosyl hydrolases, cellulase, hemicellulase, mannanase, xyloglucanse, xylanase, glucanase, glucosidase, arabinase, amylase, fructanase, laminarase, hydrolase, protease, or any combination thereof. In one embodiment, said pretreating in step ii) comprises contacting said GC rich microorganism with at least one enzyme, preferably an enzyme mix, e.g. an enzyme mix produced by an enzyme-producing microorganism selected from filamentous fungi and bacteria. In one embodiment, said pretreating in step ii) comprises a treatment of said microorganism with a hydrolase alone, or a hydrolase in combination with/followed by a protease. Optionally, said hydrolase is selected from hydrolases produced by a fungus, preferably a filamentous fungus, more preferably a fungus selected from the *Trichoderma sp., Aspergillus sp., Penicillium sp., Aureobasillium sp.* and *Fusarium sp.,* even more preferably *Trichoderma reesei.* Optionally, said protease is selected from proteases produced by *Aspergillus sp., Streptomyces sp.* or *Bacillus sp.*

In one embodiment, said at least one enzyme, e.g. enzyme mix and/or hydrolase, used for pretreating said microorganism is/are obtained from a fungus that has been cultured in the presence of an inducing system, wherein, preferably, said inducing system is a component of said GC rich microorganism, more preferably one or several cell-wall components of said GC rich microorganism, so as to obtain an enzyme mix and/or a hydrolase preparation that allows a lysis of said GC rich microorganism's cell wall. In one embodiment, said at least one enzyme, e.g. enzyme mix and/or hydrolase, preferably at least one enzyme obtained from said fungus, is prepared separately (from performing step ii) of the present invention) and is used in step ii) as a liquid preparation directly obtained from culturing said fungus, or as a freeze-dried preparation that is subsequently reconstituted in solution to be used in step ii). In one embodiment, said at least one enzyme, preferably enzyme mix, contains one or several activities for example, but not limited to enzyme activities selected from glycosyl hydrolases, cellulase, hemicellulase, mannanase, xyloglucanse, xylanase, glucanase, glucosidase, arabinase, amylase, fructanase, laminarase, hydrolase, protease, or any combination thereof.

The term "at least one enzyme", as used herein, relates to one or more enzymes, preferably an enzyme mix. In one embodiment, said at least one enzyme is capable of spheroplasting said GC rich microorganism. In one embodiment, said at least one enzyme, preferably said enzyme mix, comprises one or more enzymes selected from glycosyl hydrolases, cellulase, hemicellulase, mannanase, xyloglucanse, xylanase, glucanase, glucosidase, arabinase, amylase, fructanase, laminarase, hydrolase, protease, and any combination thereof. In one embodiment, said at least one enzyme is an enzyme mix. In one embodiment, an enzyme mix comprises hydrolase and protease, optionally further comprises further enzymes. In one embodiment, an enzyme mix comprises at least two or three, preferably at least five, more preferably at least six, even more preferably at least seven enzymes. In one embodiment, said at least one enzyme is adapted to said GC rich microorganism, i.e. by contacting said filamentous fungus with at least one component of said GC rich microorganism as an inducing system, said filamentous fungus produces enzymes specifically acting on said GC rich microorganism.

In one embodiment, the term "enzymatic pretreatment", as used herein, relates to treating a GC rich microorganism, such as an oleaginous microorganism, with at least one enzyme, preferably an enzyme mix. In one embodiment, an enzymatic pretreatment comprises contacting the GC rich microorganism with said at least one enzyme, preferably an enzyme mix. In one embodiment, such enzymatic pretreatment results in the GC rich microorganism being present in the form of a spheroplast. In one embodiment, such enzymatic pretreatment allows for an efficient transfection of said GC rich microorganism, such as an efficient transfection of an oleaginous microorganism. In one embodiment, the enzymatic pretreatment comprises contacting said GC rich microorganism with at least one enzyme selected from glycosyl hydrolases, cellulase, hemicellulase, mannanase, xyloglucanse, xylanase, glucanase, glucosidase, arabinase, amylase, fructanase, laminarase, hydrolase, protease, or any combination thereof, e.g. a hydrolase alone, or a hydrolase in combination with/followed by a protease. Optionally, said hydrolase is selected from hydrolases produced by a fungus, preferably a filamentous fungus, more preferably a fungus selected from the *Trichoderma sp., Aspergillus sp., Penicillium sp., Aureobasillium sp.* and *Fusarium sp.,* even more preferably *Trichoderma reesei;* and/or said protease is selected from proteases produced by *Aspergillus sp., Streptomyces sp.* or *Bacillus sp.*

In one embodiment, said pretreating in step ii) is performed at a temperature in the range of from 16 °C to 60 °C. In one embodiment, said pretreating in step ii) is performed at a pH in the range of from pH 3 to pH 11. In one embodiment, said pretreating in step ii) is performed under agitation, preferably under stirring with a rotational speed in the range of from 0,5 to 120 rpm. In one embodiment, said pretreating in step ii) comprises treating said GC rich microorganism with at least one enzyme, preferably an enzyme mix, for 10 min to 72 h, at a temperature in the range of from 16 °C to 60°C, in medium comprising a buffer system, and/or at a pH in the range of from pH 3 to pH 11. In one embodiment, the buffer system comprises any of water, tripotassium phosphate, citrate buffer, MES buffer, HEPES buffer, and any combination thereof.

In one embodiment, said at least one enzyme is produced by a microorganism selected from filamentous fungi and bacteria, preferably filamentous fungi. In one embodiment, the microorganism selected from filamentous fungi and bacteria is capable of producing at least one enzyme, preferably an enzyme mix, adapted to the GC rich microorganism, e.g. adapted to digest the cell wall of the GC rich microorganism. In one embodiment, the microorganism producing at least one enzyme is selected from filamentous fungi and bacteria, wherein filamentous fungi are selected from *Ceratocystis* sp., e.g. *Ceratocystis fimbriata, Ceratocystis moniliformis,* and *Ceratocystis paradoxa,* preferably *Ceratocystis paradoxa; Trichoderma* sp., e.g. *Trichoderma reesei* and *Trichoderma harzianum,* preferably *Trichoderma reesei; Aspergillus* sp., e.g. *Aspergillus oryzae, Aspergillus tubingensis,* and *Aspergillus niger; Neurospora* sp., e.g. *Neurospora intermedia; Monascus* sp., e.g. *Monascus purpureus; Rhizopus* sp., e.g. *Rhizopus oryzae; Fusarium* sp., e.g. *Fusarium venenatum; Thermomyces* sp.; *Penicillium* sp.; *Aureobasillium* sp.; *Ischnoderma* sp., e.g. *Ischnoderma benzoinum; Polyporus* sp., e.g. *Polyporus durus; Pycnoporus* sp., e.g. *Pycnoporus cinnabarinus; Phanerochaete* sp., e.g. *Phanerochaete chrysosporium;* and *Xylaria* sp; preferably selected from *Ceratocystis* sp., *Trichoderma* sp., *Aspergillus* sp., and *Fusarium* sp; more preferably selected from *Trichoderma* sp., e.g. *Trichoderma reesei;* and wherein said bacteria are selected from *Clostridium* sp., *Halobacillus* sp., *Halomonas* sp., *Rhodothermus* sp., *Streptomyces* sp., and *Bacillus* sp. In a preferred embodiment, the microorganism producing said at least one enzyme is selected from *Trichoderma* sp., preferably *Trichoderma reesei.*

In one embodiment, for obtaining said at least one enzyme for pretreating said GC rich microorganism, said enzyme-producing microorganism selected from filamentous fungi and bacteria, e.g. *Trichoderma reesei,* is cultivated with said GC rich microorganism, or a component thereof, as an inducing system. In one embodiment, said cultivating said enzyme-producing microorganism with said inducing system is performed at a temperature in the range of from 16 °C to 60 °C, preferably in the range of from 20 °C to 50 °C, more preferably in the range of from 25 °C to 40 °C, e.g. at about 30°C. In one embodiment, said cultivating said enzyme-producing microorganism is performed at a pH in the range of from pH 3 to pH 11. In one embodiment, said cultivating said enzyme-producing microorganism is performed for 1 min to 12 days, preferably for 10 min to 72 h. In one embodiment, said cultivating said enzyme-producing microorganism is performed in a medium comprising water, tripotassium phosphate, cited buffer, and/or MES buffer. In one embodiment, said cultivating said enzyme-producing microorganism is performed with p02 > 20%. In one embodiment, said cultivating said enzyme-producing microorganism with said GC rich microorganism comprises cultivating said enzyme-producing microorganism selected from filamentous fungi and bacteria with said GC rich microorganism, and thereby allowing said enzyme-producing microorganism to produce at least one enzyme.

In one embodiment, when cultivating the enzyme-producing microorganism selected from filamentous fungi and bacteria, e.g. *Trichoderma reesei,* with said GC rich microorganism or a component thereof to obtain said at least one enzyme, said GC rich microorganism, e.g. oleaginous microorganism, is nutrient limited or nutrient non-limited. For example, the GC rich microorganism, e.g. oleaginous microorganism, is cultivated under non-limiting conditions or under oil forming conditions. In one embodiment, prior to said cultivating the enzyme-producing microorganism with said GC rich microorganism, the GC rich microorganism is subjected to a cultivation under non-limiting conditions or under oil forming conditions. By cultivating the enzyme-producing microorganism with a GC rich microorganism, e.g. oleaginous microorganism, subjected to non-limiting conditions or oil forming conditions, at least one enzyme adapted to the cell wall of the GC rich microorganism in non-limiting conditions or at least one enzyme adapted to the cell wall of the GC rich microorganism in oil forming conditions, respectively, is produced.

In one embodiment, the at least one enzyme produced by said enzyme-producing microorganism selected from filamentous fungi and bacteria is suitable to hydrolyze the GC rich microorganism as a carbohydrate source. In one embodiment, said at least one enzyme produced comprises a glycosyl hydrolase, cellulase, hemicellulase, mannanase, xyloglucanse, xylanase, glucanase, glucosidase, arabinase, amylase, fructanase, laminarase, hydrolase, protease, or any combination thereof, preferably comprises a combination of cellulase, hemicellulase, mannanase, xyloglucanse, xylanase, glucanase, and glucosidase. In one embodiment, said pretreating in step ii) comprises pretreating said GC rich microorganism under limiting or non-limiting conditions. In one embodiment, if the at least one enzyme is produced using a nutrient limited GC rich microorganism, said pretreating in step ii) comprises pretreating said GC rich microorganism under limiting conditions. In one embodiment, if the at least one enzyme is produced using a nutrient non-limited GC rich microorganism, said pretreating in step ii) comprises pretreating said GC rich microorganism under non-limiting conditions. In one embodiment, under non-limiting conditions, the microorganism is fed with all nutrients required. In one embodiment, under oil forming conditions, the cultivation medium is limited in nitrogen, sulfur, and/or phosphorus.

In one embodiment, said method comprises a step of obtaining said at least one enzyme and pretreating said GC rich microorganism with said at least one enzyme to provide a spheroplast of said GC rich microorganism. In one embodiment, said pretreating comprises contacting said GC rich microorganism with said at least one enzyme, e.g. in the form of a liquid enzyme preparation obtained, preferably directly obtained, from culturing said enzyme-producing microorganism with said inducing system, or in the form of a freeze-dried enzyme preparation, optionally a freeze-dried enzyme preparation reconstituted in solution. In one embodiment, obtaining said at least one enzyme comprises obtaining said at least one enzyme in the form of liquid or in the form of a freeze-dried enzyme preparation. In one embodiment, such freeze-dried enzyme preparation of said at least one enzyme is dissolved prior to said pretreating a second substrate with said at least one enzyme.

In one embodiment, an enzymatic pretreatment is performed using at least one enzyme selected from glycosyl hydrolases, cellulase, hemicellulase, mannanase, xyloglucanse, xylanase, glucanase, glucosidase, arabinase, amylase, fructanase, laminarase, hydrolase, protease, or any combination thereof, e.g. a combination of cellulase, hemicellulase, mannanase, xyloglucanse, xylanase, glucanase, and glucosidase. In one embodiment, the enzymatic pretreatment comprises contacting said GC rich microorganism with said at least one enzyme at a temperature in the range of from 16°C to 60°C, preferably 20°C to 50°C, more preferably in the range of from 25 °C to 40 °C, e.g. for 10 min to 72 h. In one embodiment, the enzymatic pretreatment comprises contacting said GC rich microorganism with said at least one enzyme in a medium comprising a buffer, preferably having a pH in the range of from pH 3 to pH 11. In one embodiment, the buffer comprises or consists of a potassium phosphate buffer, citrate buffer and/or MES buffer. In one embodiment, the enzymatic pretreatment comprises contacting said GC rich microorganism with said at least one enzyme, e.g. selected from glycosyl hydrolases, cellulase, hemicellulase, mannanase, xyloglucanse, xylanase, glucanase, glucosidase, arabinase, amylase, fructanase, laminarase, hydrolase, protease, or any combination thereof, preferably a combination of cellulase, hemicellulase, mannanase, xyloglucanse, xylanase, glucanase, and glucosidase, such as at a temperature in the range of from 16°C to 60°C, preferably in the range of from 20°C to 50°C, more preferably in the range of from 25 °C to 40 °C, for 10 min to 72 h.

In one embodiment, the term "chemical pretreatment", as used herein, relates to treating a GC rich microorganism, e.g. an oleaginous microorganism, with at least one chemical. In one embodiment, such chemical pretreatment makes said GC rich microorganism amenable for transfection, e.g. by partially or fully removing the cell wall of said GC rich microorganism or by permeabilizing the cell wall to facilitate passage of nucleic acids and proteins. For example, by fully or partially removing the cell wall of the GC rich microorganism using a chemical, such as lithium acetate, the permeability of the GC rich microorganism is increased, and the transfer of nucleic acids, such as DNA or RNA, and proteins into the GC rich microorganism is facilitated. In one embodiment, the chemical pretreatment is a pretreatment with lithium acetate, $CaCl_2$, 2-mercaptoethanol, EDTA, dithiothreitol (DTT), DMSO, ethanol, or any combination thereof. In one embodiment, such chemical pretreatment, e.g. using lithium acetate, is performed at a temperature in the range of 16°C to 60°C, preferably 25°C to 50°C. In one embodiment, such chemical pretreatment, e.g. using lithium acetate, has a duration of from 30 sec to 24 hours.

The term "another spheroplasting procedure", as used herein, relates to a method of spheroplasting a GC rich microorganism, such as an oleaginous microorganism, other than the above mentioned enzymatic or chemical pretreatment, for example a mechanical pretreatment.

The terms "transforming" and "transformation", as used herein, relates to a genetic alteration of a cell resulting from the direct uptake and incorporation of exogenous genetic material from its surroundings through the cell membrane(s), for example, an introduction of nucleic acids, e.g. DNA and RNA, into cells. Such introduction of foreign nucleic acid using various chemical, biological, or physical methods can be used to change of the properties of the cell, e.g. allowing to modify gene function and protein expression of the cell. In one embodiment, the term "transformation" relates to any form of gene transfer such as any artificial introduction of foreign nucleic acid into a cell, e.g. transfection or transduction. In a preferred embodiment, transforming said GC rich microorganism, e.g. oleaginous microorganism, comprises or consists of transfecting said GC rich microorganism. In one embodiment, the terms "transformation" and "transfection" are used interchangeably. In one embodiment, the terms "transforming" and "transfecting" are used interchangeably. Transfection is the process of deliberately introducing nucleic acids into cells such as cells of GC rich microorganisms. The two main purposes of transfection are to produce recombinant proteins, or to specifically enhance or inhibit gene expression in transfected cells, e.g. to specifically enhance the production of acetyl-CoA-based hydrophobic compounds or specific fatty acids in GC rich microorganisms such as oleaginous microorganisms.

In one embodiment, said transforming in step iii) comprises transferring a RNA-guided endonuclease, at least one guide RNA, and optionally a donor DNA into said GC rich microorganism. In a preferred embodiment, said transforming in step iii) comprises transferring a RNA-guided endonuclease and more than one, preferably at least two or three guide RNAs, optionally a donor DNA, into said GC rich microorganism. When referring to "more than one" guide RNA, e.g. "at least two or three" guide RNAs, more than one type of guide RNA and at least two or three types of guide RNA are meant. A "type" of guide RNA relates to a guide RNA having a certain sequence; i.e. if there are "at least two" guide RNAs, such "at least two" guide RNAs comprise a first guide RNA and a second guide RNA, wherein a sequence of said first guide RNA is different from a sequence of said second guide RNA. In one embodiment, said first and second guide RNAs target opposite DNA strands on a genome of said GC rich microorganism. The inventors have surprisingly found that, when using more than one guide RNA, e.g. at least two guide RNAs, the specificity and efficiency of the transformation is highly increased. The inventors have found that such use of more than one guide RNA is particularly useful in transforming microorganisms having a high guanine-cytosine (GC) content of at least 50%, preferably at least 55%, more preferably at least 58%, even more preferably at least 60%, e.g. about 61%, since the use of more than one guide RNA reduces off-target effects. Typically, the transformation of GC rich microorganisms, e.g. oleaginous microorganisms having a GC content of at least 50 or 60%, is problematic, as primers having a high melting temperature are necessary for performing PCRs. G-C base pairs have 3 hydrogen bonds, while A-T base pairs have two; thus, double-stranded DNA with a higher number of G-C base pairs is more strongly bonded together, more stable, and has a higher melting temperature. Furthermore, the primers used for GC rich microorganisms, e.g. oleaginous microorganisms having a GC content of at least 50 or 60%, typically form secondary structures which can lead to poor or no yield of the product. Furthermore, repetitive sequences in GC rich microorganisms, e.g. oleaginous microorganisms having a GC content of at least 50 or 60%, result in off-target effects. The inventors have surprisingly found that such obstacles can be overcome by using more than one guide of RNA when transforming a GC rich microorganism such as an oleaginous microorganism, e.g. in step iii) of a method of the invention. In a preferred embodiment, the method the invention comprises transforming said GC rich microorganism with two or more guide RNAs, e.g. two or more sgRNAs having distinct sequences. Using two gRNAs even further increases the fidelity of specifically targeting the region of interest.

In one embodiment, said transforming in step iii) comprises applying said RNA-guided endonuclease in the form of a RNA-guided endonuclease protein, in the form of a DNA encoding said RNA-guided endonuclease, or in the form of a mRNA encoding said RNA-guided endonuclease to said GC rich microorganism, preferably in the form of a mRNA encoding said RNA-guided endonuclease. In one embodiment, said transforming in step iii) comprises applying said RNA-guided endonuclease and said at least one guide RNA, preferably jointly, in the form of a ribonucleoprotein complex. Advantageously, off-target activity can be reduced by application of said RNA-guided endonuclease in the form of a ribonucleoprotein complex. In one embodiment, said transforming in step iii) comprises applying said RNA-guided endonuclease in the form of a mRNA encoding said RNA-guided endonuclease, and wherein said at least one guide RNA comprises sgRNA. In one embodiment, when referring to at least one guide RNA "comprising" sgRNA, said at least one guide RNA comprises or consists of sgRNA. In one embodiment, sgRNA comprises or consists of crRNA and tracrRNA. In a preferred embodiment, said transforming in step iii) comprises transforming said GC rich microorganism with a RNA guided endonuclease in the form of mRNA encoding said RNA-guided endonuclease. In a preferred embodiment, said transforming in step iii) comprises transforming said GC rich microorganism with more than one guide RNA, preferably at least two or three guide RNAs. In one embodiment, said at least one guide RNA comprises a first guide RNA and a second guide RNA, wherein a sequence of said first guide RNA is different from a sequence of said second guide RNA. In one embodiment, said at least two or three guide RNAs comprise a first guide RNA and a second guide RNA, and a third guide RNA in a case of said at least three guide RNAs, wherein a sequence of said first guide RNA is different from a sequence of said second guide RNA, and, if present, different from a sequence of said third guide RNA.

In one embodiment, said transforming in step iii) is performed using electroporation; PEG-based or other nanoscale carrier-based transformation; biological ballistics; glass bead transformation; vesicle-mediated delivery; a viral transfection system, e.g. lentivirus (LVs), adenovirus (AdV), or adeno-associated virus (AAV); liposomal delivery, e.g. lipofection; a chemical transfection technique; or combinations thereof. In a preferred embodiment, said transforming in step iii) is performed using electroporation and/or PEG-based transformation, preferably using electroporation. In one embodiment, said transforming in step iii) comprises a targeted genetic modification of a chromosome or episome of said GC rich microorganism, preferably GC rich oleaginous microorganism. In one embodiment, a PEG-based transformation comprises PEG promoting an association of the nucleic acid (the nucleic acid to be transferred into a cell) with the surface of the cell. Optionally, said PEG-based transformation further comprises lithium ions and heat shock promoting passage of DNA into the cell. In one embodiment, a nanoscale carrier-based transformation is a polymer-based transformation and/or a nanoparticle-based transformation, e.g. comprising carriers such as carrier DNA, poly-DMAEMA carriers, and BG2 carriers. In one embodiment, when said transforming in step iii) comprises applying said RNA-guided endonuclease and said at least one guide RNA in the form of a ribonucleoprotein complex, said ribonucleoprotein complex is applied using vesicle-mediated delivery. In one embodiment, when referring to "applying" in the context of transformation, such term is meant to relate to transferring and/or administering a compound, such as a RNA-guided endonuclease and at least one guide RNA.

The term "RNA-guided endonuclease", as used herein, relates to any RNA guided endonuclease known to a person skilled in the art. In one embodiment, said RNA-guided endonuclease is a CRISPR-associated (Cas) endonuclease, preferably selected from Cas9, Cas1, Cas2, Cas4, Cas3, Cas10, Casi2, Cas13, Csm, scfi, or variants thereof, e.g. Casi2a, dCas9, D10A CAS9 nickase or H840A CAS9 nickase. A "variant" of an RNA-guided endonuclease relates to an RNA-guided endonuclease which has been artificially modified, e.g. by mutation such as point mutation, to comprise a desired feature such a modified cleavage site only cleaving a single-strand rather than a double-strand, or which naturally comprises a modification due to its origin from a different organism, e.g. RNA-guided endonuclease analogs, homologues, and orthologues. For example, a variant of Cas9 is dCas9, D10A CAS9 nickase or H840A CAS9 nickase, and a variant of Casi2 is Casi2a. In one embodiment, when referring to an endonuclease e.g. Cas9, said endonuclease obtained from any microorganism is meant, e.g. Cas9 isolated from *Streptococcus pyogenes* (SpCas9), *Staphylococcus aureus* (SaCas9), *Streptococcus thermophilus* (StCas9), *Streptococcus canis* (ScCas9), *Neisseria meningitidis* (NmCas9), *Francisella novicida* (FnCas9), or *Campylobacter jejuni* (CjCas9). In one embodiment, the RNA-guided endonuclease is used in a method of the invention, e.g. in step iii), in the form of a RNA-guided endonuclease protein, in the form of a DNA encoding said RNA-guided endonuclease, or in the form of a mRNA encoding said RNA-guided endonuclease, preferably in the form of a mRNA encoding said RNA-guided endonuclease. In one embodiment, said transforming in step iii) comprises applying is said RNA-guided endonuclease in the form of mRNA and said at least one guide RNA, preferably more than one guide RNA, in the form of sgRNA or in the form of crRNA and tracrRNA. An advantage of the method of the invention is that the RNA-guided endonuclease can be applied in the form of mRNA and the at least one guide RNA can be applied in the form of sgRNA or in the form of crRNA and tracrRNA, thereby providing a highly efficient method with high transformation yield. Furthermore, using more than one type of guide RNA, i.e. at least two guide RNAs having distinct sequences, such as at least two sgRNAs having distinct sequences, enhances the target specificity for the microorganisms with repeated and GC rich sequences in their genome, and hence lower the frequency of the off-target activities is observed. Accordingly, an advantage of the method of genetically modifying a GC rich microorganism is that a GC rich microorganism, for example an oleaginous microorganism having a high GC content, can be efficiently transformed despite the abundance of repetitive sequences. In one embodiment, the RNA-guided endonuclease is Cas9, e.g. Cas9 comprising a sequence of any of SEQ ID NO. 1-4.

The term "guide RNA" or "gRNA", as used herein, relates to an RNA that functions as a guide for RNA- or DNA-targeting enzymes, e.g. by forming a complex with an RNA- or DNA-targeting enzyme such as an RNA-guided endonuclease, e.g. sgRNA. In one embodiment, the at least one guide RNA comprises any of CRISPR RNA (crRNA), trans-activating CRISPR RNA (tracrRNA), and single-guide RNA (sgRNA), preferably comprises a) sgRNA and/or b) crRNA and tracrRNA. In one embodiment, the at least one guide RNA comprises or consists of crRNA and tracrRNA, and/or comprises or consists of sgRNA. The term "at least one guide RNA", as used herein, relates to one or more guide RNAs, preferably one or more types of guide RNAs, e.g. guide RNAs having distinct sequences. In one embodiment, if said at least one guide RNA comprises two or more guide RNAs, a sequence of a second or further guide RNA is different from a sequence of a first guide RNA. Furthermore, a sequence of said further guide RNA is different from a sequence of said first and second guide RNAs. In one embodiment, said transforming in step iii) comprises transferring one or more molecules, preferably several molecules, of each of said at least one guide RNA, e.g. of each of said one or more types of guide RNA, to said GC rich microorganism. Advantageously, when using at least two types of guide RNAs, the efficiency of the transformation of a method of the invention can be even further increased. In one embodiment, said at least one guide RNA comprises or consists of CRISPR RNA (crRNA), trans-activating CRISPR RNA (tracrRNA), and/or single-guide RNA (sgRNA). In a preferred embodiment, said at least one guide RNA comprises or consists of sgRNA. In one embodiment, said sgRNA comprises a crRNA and a tracrRNA. In a preferred embodiment, said at least one guide RNA comprises a first guide RNA and a second guide RNA, wherein a sequence of said first guide RNA is different from a sequence of said second guide RNA. In one embodiment, tracrRNA comprises or consists of a sequence having SEQ ID NO. 5.

In one embodiment, the term "donor DNA", as used herein, relates to any DNA of interest to be transferred into said GC rich microorganism, preferably incorporated into a genome of said GC rich microorganism. In one embodiment, donor DNA comprises a DNA repair template, a DNA encoding a selection marker, and/or a gene or sequence of interest, e.g. a gene involved in the production of a target compound such as acetyl-CoA or an acetyl-CoA-based hydrophobic compound or a fatty acid. For example, when using a DNA repair template, a specific mutation, e.g. a point mutation and/or knockout, can be incorporated into the genome of said GC rich microorganism. In one embodiment, the gene or sequence of interest encodes delta-9-desaturase, delta-12-desaturase, elongase, oleat hydratase, aldo keto reductase promoter, aldo keto reductase terminator, transcription elongation factor 2 promoter, TEF promoter, and/or TEF terminator. In one embodiment, donor DNA is single-stranded or double-stranded. In one embodiment, a donor DNA comprises or consists of a sequence of any of SEQ ID NO. 6-13 and 27, and/or of a sequence having at least 80%, preferably at least 90% or at least 99% sequence identity with a sequence of any of SEQ ID NO. 6-13 and 27. In one embodiment, the donor DNA comprises a DNA encoding for a gene involved in any of i) cellular processes and signaling, such as involved in cell wall/membrane/envelope biogenesis, cell motility, posttranslational modification, protein turnover, chaperones, signal transduction mechanisms, intracellular trafficking, secretion, vesicular transport, defense mechanisms, extracellular structures, nuclear structure, and/or cytoskeleton; ii) information storage and processing, such as involved in RNA processing and modification, chromatin structure and dynamics, translation, ribosomal structure and biogenesis, transcription, and/or replication, recombination and repair; iii) metabolism, such as involved in energy production and conversion, cell cycle control, cell division, chromosome partitioning, amino acid transport and metabolism, nucleotide transport and metabolism, carbohydrate transport and metabolism, coenzyme transport and metabolism, lipid transport and metabolism, inorganic ion transport and metabolism, and/or secondary metabolites biosynthesis, transport and catabolism.

In one embodiment, the term "selecting a transformed cell", as used herein, relates to selecting the GC rich microorganisms which have been successfully transformed. The GC rich microorganism has been successfully transformed if the GC rich microorganism comprises said RNA-guided endonuclease, said at least one guide RNA, and optionally said donor DNA; preferably if said GC rich microorganism has been genetically modified e.g. by a DNA cleavage cleaved by said RNA-guided endonuclease and/or by incorporation of said donor DNA, if present, into a genome of said GC rich microorganism. In one embodiment, said selecting a transformed cell in step iv), if present, comprises selecting said transformed GC rich microorganism, e.g. a transformed oleaginous microorganism, using a selection marker and/or a selective agent. In one embodiment, said method comprises said selecting in step iv), wherein said selecting in step iv) comprises subjecting said microorganism to a selective agent selective for said selection marker, optionally a selection marker encoded by said donor DNA. In one embodiment, said selecting comprises subjecting said microorganism to a selective agent at a concentration in the range of from 10 % to 90 %, preferably 10 % to 70 %, more preferably 10 % to 60 %, of the minimum selectable concentration; and/or subjecting said microorganism to a selective agent at a concentration in the range of from 90 % to 100 %, preferably 99 % to 100 %, of the minimum selectable concentration.

In one embodiment, said selecting in step iv), if present, is a two-step selection, wherein, in a first step, said GC rich microorganism is subjected to a medium having a lower selection strength than a medium of a second step, and in a second step, said GC rich microorganism is subjected to a medium having a higher selection strength than a medium of the first step. In one embodiment, said selecting in step iv), if present, is a two-step selection wherein, in a first step, said GC rich microorganism is subjected to an upper layer of agar having a lower selection strength than a lower layer of agar, and in a second step, said GC rich microorganism is subjected to a lower layer of agar having a higher selection strength than the upper layer of agar. In a preferred embodiment, said upper layer of agar and said lower layer of agar are arranged in one plate on top of each other. In one embodiment, said selecting in step iv), if present, comprises subjecting said GC rich microorganism to an upper layer of agar comprising a selection strength lower than a selection strength of a lower layer of agar, wherein, if said GC rich microorganism is successfully transformed, said GC rich microorganism grows on said upper layer. In one embodiment, when said GC rich microorganism growing on said upper layer further grows towards said lower layer, preferably towards and on said lower layer, said lower layer having a higher selection strength than the upper layer, said GC rich microorganism is a successfully transformed GC rich microorganism.

In one embodiment, selection strength relates to the presence of a certain concentration of a selective agent, e.g. a selection strength of 60% relates to the presence of a selective agent at a concentration of 60% of the minimum selectable concentration or a concentration of 60% of the minimum lethal concentration of said selective agent. In one embodiment, said selecting comprises subjecting said microorganism to said selective agent at a concentration in the range of from 10 % to 90 %, preferably 10 % to 70 %, more preferably 10 % to 60 %, of the minimum selectable concentration in a first medium, e.g. a liquid medium or solid medium, preferably in an upper layer of agar, and subsequently subjecting said microorganism to a selective agent at a concentration in the range of from 90 % to 100 %, preferably 99 % to 100 % in a second medium, e.g. a liquid medium or solid medium, preferably in a lower layer of agar. In one embodiment, said selecting in step iv) comprises a two-step selection, i.e. said selecting comprises subjecting said microorganism to a first agar and a second agar. Such two-step selection is advantageous in that the first agar with a lower concentration of selective agent allows for a regeneration of the microorganisms, preferably spheroplasts, in an environment where the concentration of the selective agent is lower than in the second agar, e.g. lower but in the selectable range, such as lower than $IC_{50}$ (such as of compounds with toxicity effects, e.g. 5-FOA). Such regeneration of the microorganisms is especially advantageous for the microorganisms which are subjected to a transformation in a subsequent step, e.g. microorganisms which obtain the intended gene editing/selectable marker gene in a subsequent step. In one embodiment, the term "minimum selectable concentration", as used herein, relates to a concentration of an agent, e.g. selective agent, at which a visible or measurable selection is possible. In one embodiment, the minimum selectable concentration relates to the minimum inhibitory concentration. In one embodiment, the minimum selectable concentration is determined by preparing plates with serial dilutions of the selective agent and observing the growth of the microorganism.

In one embodiment, the term "genetically modified GC rich microorganism", as used herein, relates to a GC rich microorganism, e.g. oleaginous microorganism, that has been transformed and/or transfected, preferably a GC rich microorganism genetically modified using a method of the present invention. For example, the genetic material of a genetically modified GC rich microorganism has been altered using genetic engineering. In one embodiment, a genetically modified GC rich microorganism has a GC content of at least 50%, preferably at least 55%, more preferably at least 58%, even more preferably at least 60%, e.g. about 61%. In one embodiment, a genetically modified GC rich microorganism is a genetically modified oleaginous microorganism such as oleaginous yeast, preferably selected from *Cutaneotrichosporon* sp., more preferably *Cutaneotrichosporon oleaginosus.* In one embodiment, a genetically modified GC rich microorganism comprises an RNA-guided endonuclease, at least one guide RNA, and optionally a donor DNA. In one embodiment, said RNA-guided endonuclease and said at least one guide RNA are present in said cell in the form of a ribonucleoprotein complex. In one embodiment, said method further comprises a step of sequencing the genome of the genetically modified GC rich microorganism obtained in step v).

In one embodiment, the term "genetically modified oleaginous microorganism", as used herein, relates to an oleaginous microorganism that has been transformed and/or transfected, preferably an oleaginous microorganism genetically modified using a method of the present invention. For example, the genetic material of a genetically modified oleaginous microorganism has been altered using genetic engineering. In one embodiment, a genetically modified oleaginous microorganism is an oleaginous microorganism having a GC content of at least 50%, preferably at least 55%, more preferably at least 58%, even more preferably at least 60%. In one embodiment, a genetically modified oleaginous microorganism is a genetically modified oleaginous yeast, preferably selected from *Cutaneotrichosporon* sp., more preferably *Cutaneotrichosporon oleaginosus.* In one embodiment, a genetically modified oleaginous microorganism comprises a RNA-guided endonuclease, at least one guide RNA, and optionally a donor DNA. In one embodiment, said RNA-guided endonuclease and said at least one guide RNA are present in said cell in the form of a ribonucleoprotein complex. In a preferred embodiment, the genetically modified GC rich microorganism of the present invention has a GC content of at least 50%, preferably at least 55%, more preferably at least 58%, even more preferably at least 60%, e.g. about 61%.

The term "selective agent", as used herein, relates to an agent which affect an organism's ability to survive in a given environment, for example an antibiotic. For example, after successful transfection, the GC rich microorganism may comprise a selection marker, such that successfully transfected cells can be selected using a selective agent specific for said selection marker. For example, a selective agents such as an antibiotic and allows to specifically select transfected cells comprising the respective antibiotic resistance gene. In one embodiment, the selective agent used in a method of the invention is selected from 5-fluoroorotic acid, 5-fluoroanthranilic acid, hygromycin B, geneticin, phleomycin, and any combination thereof.

In one embodiment, the term "selection marker", as used herein, relates to a nucleic acid sequence, e.g. gene, or compound comprised by a cell that confers a trait suitable for selection. In one embodiment, a selection marker is a gene, which is to be introduced into a cell, that confers a trait suitable for artificial selection. Typically, selection markers indicate the success of a transformation, transfection or any procedure meant to introduce foreign DNA into a cell. Selection markers can be antibiotic resistance genes. For example, a selection marker can be a reporter gene and/or antibiotic resistance gene to indicate the success of a transfection or other procedure meant to introduce foreign DNA into a cell. In a preferred embodiment, the selection marker is an antibiotic resistance gene. The GC rich microorganisms having been subjected to transforming in step iii) can be grown on a medium containing the corresponding selective agent, e.g. said antibiotic, and those colonies that can grow have successfully taken up and expressed the introduced genetic material, e.g. donor DNA. In one embodiment, the selection marker is a positive or negative selection marker. In one embodiment, the selection marker is encoded by said donor DNA. In one embodiment, the selection marker is selected from marker genes which encode essential enzymes for de novo nucleic acid and amino acid synthesis, marker genes involved in nutrient utilizations such as acetamidase, and antimicrobial resistance genes. In one embodiment, the selection marker is selected from URA5, URA3, HIS, LEU, TRP, MET, ADE, LYS, URA, ARG, ALA, sulfonylurea, sulfometuron, acetamide, formamide, hygromycin B resistance gene, KanMx resistance gene, zeocin resistance gene, nourseothricin, phleomycin, puromycin, aureobasidin, cycloheximide, and erythromycin. Furthermore, also endogenous genes can be used as selection marker; for example a compound or drug can be toxic to an untransformed GC rich microorganism due to the expression of a particular endogenous gene, while the transformed cells will survive since they lack the ability to form toxic compounds. Mutations created on these target genes can be selected by plating the transformants on medium supplemented with the appropriate inhibitors. In one embodiment, the selection marker is introduced into said cell via transformation, e.g. as a part of the donor DNA, or is endogenous in said cell.

In one embodiment, the term "DNA repair template", as used herein, relates to DNA which comprises a DNA sequence of interest, for example a fragment of a region of genomic DNA centered around a location of a desired modification. For example, such DNA repair template can be used to incorporate a genetic modification, such as a point mutation in the genome of an organism. In one embodiment, the DNA repair template comprises a sequence of the genome of the GC rich microorganism, wherein such sequence comprises a desired mutation. In one embodiment, the term "gene or sequence of interest", as used herein, relates to a nucleic acid of interest, such as a gene involved in the production of a target compound, such as acetyl-CoA pool or an acetyl-CoA-based hydrophobic compound or a fatty acid, a promoter or terminator involved in the production of said target compound, and/or a selection marker. In one embodiment, the gene or sequence of interest encodes a product of interest, such as a protein or enzyme. In one embodiment, the gene or sequence of interests encodes for any gene involved in i) cellular processes and signaling, such as involved in cell wall/membrane/envelope biogenesis, cell motility, posttranslational modification, protein turnover, chaperones, signal transduction mechanisms, intracellular trafficking, secretion, vesicular transport, defense mechanisms, extracellular structures, nuclear structure, and/or cytoskeleton; ii) information storage and processing, such as involved in RNA processing and modification, chromatin structure and dynamics, translation, ribosomal structure and biogenesis, transcription, and/or replication, recombination and repair; iii) metabolism, such as involved in energy production and conversion, cell cycle control, cell division, chromosome partitioning, amino acid transport and metabolism, nucleotide transport and metabolism, carbohydrate transport and metabolism, coenzyme transport and metabolism, lipid transport and metabolism, inorganic ion transport and metabolism, and/or secondary metabolites biosynthesis, transport and catabolism. In one embodiment, when referring to "a gene involved in the production of said target compound and/or specific fatty acid", a gene or sequence of interest is meant. In one embodiment, a gene or sequence of interest encodes any of delta-9-desaturase; delta-12-desaturase; elongase; oleat hydratase; aldo keto reductase promoter; aldo keto reductase terminator; transcription elongation factor 2 promoter; TEF promoter; TEF terminator; 1, 2-alpha-mannosidase; 1,4-alpha-glucan branching enzyme/starch branching enzyme II; 15-hydroxyprostaglandin dehydrogenase and related dehydrogenases; 17 beta-hydroxysteroid dehydrogenase; 1-acyl-sn-glycerol-3-phosphate acyltransferase; 2-enoyl-CoA hydratase/3-hydroxyacyl-CoA dehydrogenase/Peroxisomal 3-ketoacyl-CoA-thiolase; sterol-binding domain and related enzymes; 2-oxoglutarate dehydrogenase; 3-hydroxy-3-methylglutaryl-CoA (HMG-CoA) reductase; 3-hydroxyacyl-CoA dehydrogenase; 3-keto sterol reductase; 3-Methylcrotonyl-CoA carboxylase; Propionyl-CoA carboxylase; alpha chain/Acetyl-CoA carboxylase; 3-Methylcrotonyl-CoA carboxylase; Acetyl-CoA carboxylase carboxyl transferase; 3-oxoacyl CoA thiolase; 3-oxoacyl-(acyl-carrier-protein) synthase (I and II); 3-phosphoglycerate kinase; 5'-AMP-activated protein kinase; 5'-phosphoribosylglycinamide formyltransferase; 6-phosphogluconate dehydrogenase; 6-phosphogluconolactonase; Acetyl-CoA acetyltransferase; Acetyl-CoA carboxylase; Acetylglucosaminyltransferase EXT1/exostosin 1; Acid sphingomyelinase and PHM5 phosphate metabolism protein; Aconitase/homoaconitase (aconitase superfamily); Acyl carrier protein/NADH-ubiquinone oxidoreductase; Acyl-CoA oxidase; Acyl-CoA synthetase; Acyl-CoA thioesterase; Acyl-CoA:diacylglycerol acyltransferase (DGAT); Acyl-CoA-binding protein; Acylphosphatase; Alcohol dehydrogenase; Aldehyde dehydrogenase; Alkaline ceramidase; Alpha amylase; Alpha-1;4-N-acetylglucosaminyltransferase; Alpha-amylase; Alpha-mannosidase; Amidases; Aquaporin; ATP binding protein; ATP-citrate lyase; Beta-1,6-N-acetylglucosaminyltransferase; Beta-fructofuranosidase (invertase); Beta-glucosidase; lactase phlorizinhydrolase; and related proteins; Beta-Glucuronidase GUSB (glycosylhydrolase superfamily 2); Betaine aldehyde dehydrogenase; Beta-N-acetylhexosaminidase; Bifunctional leukotriene A4 hydrolase/aminopeptidase LTA4H; Branched chain alpha-keto acid dehydrogenase complex; C-3 sterol dehydrogenase/3-beta-hydroxysteroid dehydrogenase and related dehydrogenases; C-4 sterol methyl oxidase; Carnitine O-acyltransferase; CDP-alcohol phosphatidyltransferase/Phosphatidylglycerolphosphate synthase; CDP-diacylglycerol synthase; Ceramide glucosyltransferase; Chitinase; Cholesterol transport protein; Choline transporter-like protein; Cis-prenyltransferase; Citrate synthase; Class II aldolase/adducin N-terminal domain protein; Cytochrome b5; Cytochrome c; Cytochrome c oxidase; Cytochrome c1; Cytochrome P450 CYP4/CYP19/CYP26 subfamilies; Delta 6-fatty acid desaturase/delta-8 sphingolipid desaturase; Dihydrolipoamide acetyltransferase; Dihydrolipoamide dehydrogenase; Dihydrolipoamide succinyltransferase (2-oxoglutarate dehydrogenase; E2 subunit); Dihydrolipoamide transacylase (alpha-keto acid dehydrogenase E2 subunit); Dihydroxyacetone kinase/glycerone kinase; Dimeric dihydrodiol dehydrogenase; Dolichol kinase; Dolichyl pyrophosphate phosphatase and related acid phosphatases; D-ribulose-5-phosphate 3-epimerase; dTDP-glucose 4-6-dehydratase/UDP-glucuronic acid decarboxylase; Electron transfer flavoprotein ubiquinone oxidoreductase; Electron transfer flavoprotein; Enolase; Enoyl-CoA hydratase; Enoyl-CoA hydratase/isomerase; Enoyl-CoA isomerase; Exopolyphosphatases and related proteins; FoF1-type ATP synthase; Fi-ATP synthase assembly protein; Fatty acid desaturase; Fatty acyl-CoA elongase/Polyunsaturated fatty acid specific elongation enzyme; Ferredoxin; Flavohemoprotein b5+b5R; Fructose 1,6-bisphosphate aldolase; Fructose-1,6-bisphosphatase; Fructose-6-phosphate 2-kinase/fructose-2,6-biphosphatase; Fumarase; Fumarate reductase; Fumarylacetoacetase; Galactose-i-phosphate uridylyltransferase; Galactosyltransferases; Gamma-butyrobetaine;2-oxoglutarate dioxygenase; GDP-mannose pyrophosphorylase; Globins and related hemoproteins; Gluconate kinase; Gluconate transport-inducing protein; Glucosamine-6-phosphate isomerase; Glucose-6-phosphate 1-dehydrogenase; Glucose-6-phosphate isomerase; Glucose-6-phosphate/phosphate and phosphoenolpyruvate/phosphate antiporter; Glucosidase I; Glucosidase II catalytic (alpha) subunit and related enzymes; glycosyl hydrolase family 31; Glyceraldehyde 3-phosphate dehydrogenase; Glycerol-3-phosphate dehydrogenase; Glycerol-3-phosphate dehydrogenase/dihydroxyacetone 3-phosphate reductase; Glycerophosphoryl diester phosphodiesterase; Glycogen phosphorylase; Glycogen synthase; Glycogen synthase kinase; Glycolate oxidase; Glycolipid 2-alpha-mannosyltransferase (alpha-1,2-mannosyltransferase); Glycolipid transfer protein; Glycosyl hydrolase; Glycosyltransferase; Glyoxalase; Glyoxylate/hydroxypyruvate reductase (D-isomer-specific 2-hydroxy acid dehydrogenase superfamily); Hexokinase; Holocytochrome c synthase/heme-lyase; Hormone-sensitive lipase HSL; Hydroxyacyl-CoA dehydrogenase/enoyl-CoA hydratase; Hydroxymethylglutaryl-CoA lyase; Hydroxymethylglutaryl-CoA synthase; Inorganic pyrophosphatase; Inositol monophosphatase; Inositol phospholipid synthesis protein; Scs3p; Inositol polyphosphate multikinase; Isoamyl acetate-hydrolyzing esterase; Isocitrate lyase; Isovaleryl-CoA dehydrogenase; Kynurenine 3-monooxygenase; Lecithin:cholesterol acyltransferase (LCAT)/Acyl-ceramide synthase; Lipid exporter ABCA1 and related proteins; ABC superfamily; Lipid phosphate phosphatase and related enzymes of the PAP2 family; Lipoyltransferase; Long-chain acyl-CoA synthetases (AMP-forming); Low density lipoprotein B-like protein; Lysophosphatidic acid acyltransferase endophilin/SH3GL; Lysophosphatidic acid acyltransferase LPAAT and related acyltransferases; Lysophospholipase; Malate synthase; Maltase glucoamylase and related hydrolases; glycosyl hydrolase family 31; MAM33; mitochondrial matrix glycoprotein; Mannose-6-phosphate isomerase; Mannosyltransferase; Medium-chain acyl-CoA dehydrogenase; Methylmalonate semialdehyde dehydrogenase; Mevalonate pyrophosphate decarboxylase; Mitochondrial ADP/ATP carrier proteins; Mitochondrial aspartate/glutamate carrier protein Aralar/Citrin; Mitochondrial carnitine-acylcarnitine carrier protein; Mitochondrial carrier protein; Mitochondrial F1F0-ATP synthase; Mitochondrial FAD carrier protein; Mitochondrial Fe-S cluster biosynthesis protein ISA2; Mitochondrial oxaloacetate carrier protein; Mitochondrial oxodicarboxylate carrier protein; Mitochondrial oxoglutarate/malate carrier proteins; Mitochondrial phosphate carrier protein; Mitochondrial protein Surfeit 1/SURF1/SHY1; Mitochondrial solute carrier protein; Mitochondrial tricarboxylate/dicarboxylate carrier proteins; Mitochondrial/plastidial beta-ketoacyl-ACP reductase Monocarboxylate transporter; Monooxygenase involved in coenzyme Q (ubiquinone) biosynthesis; Myo-inositol-i-phosphate synthase; N-Acetylglucosamine kinase; N-acetyl-glucosamine-6-phosphate deacetylase; N-acetylglucosaminyltransferase complex; NAD-dependent malate dehydrogenase; NADH:flavin oxidoreductase/12-oxophytodienoate reductase; NADH:ubiquinone oxidoreductases; NADH-cytochrome b-5 reductase; NADH-dehydrogenase; NADP/FAD dependent oxidoreductase; NADP+-dependent malic enzyme; NADP-dependent flavoprotein reductase; NADP-dependent isocitrate dehydrogenase; NADPH oxidase; Neutral trehalase; N-myristoyl transferase; Nucleotide-sugar transporter; Oligoketide cyclase/lipid transport protein; O-linked N-acetylglucosamine transferase OGT; Oxidosqualenelanosterol cyclase; Oxysterol-binding protein; Palmitoyl protein thioesterase; Peroxisomal 3-ketoacyl-CoA-thiolase P-44/SCP2; Peroxisomal long-chain acyl-CoA transporter; ABC superfamily; Peroxisomal multifunctional beta-oxidation protein; Peroxisomal phytanoyl-CoA hydroxylase; Phosphate acyltransferase; Phosphatidic acid-preferring phospholipase A1; Phosphatidylglycerolphosphate synthase; Phosphatidylinositol synthase; Phosphatidylinositol transfer protein PDR16 and related proteins; Phosphatidylinositol transfer protein SEC14 and related proteins; Phosphatidylserine decarboxylase; Phosphoglucomutase; Phosphoglucomutase/phosphomannomutase; Phosphoglycerate mutase; Phosphoinositide phosphatase SACi; Phospholipase; Phospholipase A2; Phospholipase A2-activating protein; Phospholipase Di; Phospholipid methyltransferase; Phosphomannomutase; Phosphomevalonate kinase; Phosphorylcholine transferase/cholinephosphate cytidylyltransferase; Phytoene/squalene synthetase; pfkB family carbohydrate kinase; 2-oxoglutarate dehydrogenase; Beta-mannosidase; Dehydrogenase; Gamma-butyrobetaine,2-oxoglutarate dioxygenase; L-carnitine dehydratase/alpha-methylacyl-CoA racemase; Lipase/calmodulin-binding heat-shock protein; Mitochondrial carrier protein; Mitochondrial cholesterol transporter; Mutarotase; Oxidoreductase; Phosphoglucosamine acetyltransferase; Phosphoglycerate mutase; Phospholipase; Quinone oxidoreductase; Sugar kinase; Sugar transporter; UDP-galactose transporter; Pristanoyl-CoA/acyl-CoA oxidase; Prohibitins and stomatins of the PID superfamily; Proteins containing the FAD binding domain; Purple acid phosphatase; Cytochrome C oxidase assembly protein; Lipases e.g. lipase essential for disintegration of autophagic bodies inside the vacuole; NAD+-dependent epimerases; Phosphoinositide phosphatase; Trehalase; Pyrophosphate-dependent phosphofructo-i-kinase; Pyruvate carboxylase; Pyruvate dehydrogenase E1; Pyruvate kinase; Ribokinase; Ribose 5-phosphate isomerase; Ribulose kinase and related carbohydrate kinases; SAM-dependent methyltransferases; Serine/threonine kinase receptor; Serine/threonine protein kinase; Serine/threonine specific protein phosphatase; Short-chain acyl-CoA dehydrogenase; sn-1,2-diacylglycerol ethanolamine- and cholinephosphotranferases; Soluble epoxide hydrolase; Sphingoid base-phosphate phosphatase; Sphingolipid fatty acid hydroxylase; Sphingolipid hydroxylase; Sphingosine kinase; Squalene monooxygenase; Squalene synthetase; Steroid reductase; Sterol C5 desaturase; Sterol O-acyltransferase/Diacylglycerol O-acyltransferase; Sterol reductase/lamin B receptor; Succinate dehydrogenases; Succinyl-CoA synthetase; Succinyl-CoA:alpha-ketoacid-CoA transferase; Sucrose transporter and related proteins; Sugar (pentulose and hexulose) kinases; Sugar transporter/spinster transmembrane protein; Sulfatases; Sulfide:quinone oxidoreductase/flavo-binding protein; Transaldolase; Transketolase; Transthyretin and related proteins; Trehalose-6-phosphate synthase; Triglyceride lipase-cholesterol esterase; Triosephosphate isomerase; Ubiquinol cytochrome c reductase assembly protein; Ubiquinol cytochrome c reductase; UDP-galactose transporter; UDP-glucose pyrophosphorylase; UDP-glucose/GDP-mannose dehydrogenase; UDP-glucose:glycoprotein glucosyltransferase; UDP-glucuronosyl and UDP-glucosyl transferase; UDP-N-acetylglucosamine transporter; Aldose 1-epimerase; Vacuolar H+-ATPase; Very-long-chain acyl-CoA dehydrogenase; Vigilin; Voltage-gated shaker-like K+ channel; Zinc-binding oxidoreductase, and any combination thereof.

The term "oil having a specific fatty acid profile, e.g. high oleic oil", as used herein, relates to an oil having a desired fatty acid profile, e.g. comprising certain types of fatty acids such as unsaturated fatty acids. In one embodiment, a high oleic oil is an oil comprising at least 50%, preferably at least 60%, e.g. at least 70%, oleic acid.

In one embodiment, said method of genetically modifying a GC rich microorganism e.g. oleaginous microorganism and/or said pretreating in step ii) comprises:

a) growing said GC rich microorganism, e.g. oleaginous microorganism, to an OD of up to OD between 0.2 and 5.0, e.g., between 0.2x $10^7$ and 2x $10^8$ cells/mL ;
b) harvesting the cells grown in step a);
c) washing the cells harvested in step b), e.g. with sterile water followed by sorbitol (1 M);
d) treating the cells washed in step c) with said at least one enzyme, preferably in a suitable buffer;

e) harvesting the cells treated in step d);
f) resuspending the cells harvested in step e) in a suitable buffer, e.g. tris-HCl buffer containing sorbitol or mannitol and $CaCl_2$.

In one embodiment, prior to said transforming in step iii), said GC rich microorganism is grown, washed, and treated with said at least one enzyme. In one embodiment, said pretreating in step ii) comprises a step of washing said microorganism with an organic phase, e.g. methanol, ethanol, butanol, and/or amyl alcohol; a buffer; and/or water; preferably prior to a contacting of said microorganism with at least one enzyme, e.g. selected from glycosyl hydrolases, cellulase, hemicellulase, mannanase, xyloglucanse, xylanase, glucanase, glucosidase, arabinase, amylase, fructanase, laminarase, hydrolase, protease, or any combination thereof. In one embodiment, said pretreating in step ii) comprises an enzymatic pretreatment and/or another spheroplasting procedure comprising contacting said microorganism with at least one enzyme produced by an enzyme-producing filamentous fungus; wherein, optionally, said enzymatic pretreatment and/or said another spheroplasting procedure comprises washing said microorganism with an organic phase, a buffer, and/or water prior to said contacting. In one embodiment, a method of the invention comprises a step of washing said GC rich microorganism with an organic phase, a buffer, and/or water, e.g. prior to said pretreating of step ii). In one embodiment, a method of the invention comprises a step of washing said GC rich microorganism with an organic phase, a buffer, and/or water, preferably prior to said contacting said GC rich microorganism with at least one enzyme.

In one embodiment, an organic phase comprises or consists of methanol, amyl alcohol, ethanol, butanol, or any combination thereof. In one embodiment, said enzyme-producing filamentous fungus is selected from *Ceratocystis* sp., e.g. *Ceratocystis fimbriata, Ceratocystis moniliformis,* and *Ceratocystis paradoxa,* preferably *Ceratocystis paradoxa; Trichoderma* sp., e.g. *Trichoderma reesei* and *Trichoderma harzianum,* preferably *Trichoderma reesei; Aspergillus* sp., e.g. *Aspergillus oryzae, Aspergillus tubingensis,* and *Aspergillus niger; Neurospora* sp., e.g. *Neurospora intermedia; Monascus* sp., e.g. *Monascus purpureus; Rhizopus* sp., e.g. *Rhizopus oryzae; Fusarium* sp., e.g. *Fusarium venenatum; Thermomyces* sp.; *Penicillium* sp.; *Aureobasillium* sp.; *Ischnoderma* sp., e.g. *Ischnoderma benzoinum; Polyporus* sp., e.g. *Polyporus durus; Pycnoporus* sp., e.g. *Pycnoporus cinnabarinus; Phanerochaete* sp., e.g. *Phanerochaete chrysosporium;* and *Xylaria* sp; preferably produced by *Trichoderma reesei.*

In one embodiment, the at least one enzyme produced by a fungus, e.g. hydrolases or proteases produced by a fungus, are produced by growing said fungus; harvesting the cells of said fungus; optionally, washing the cells with water and/or sorbitol. In one embodiment, said pretreating in step ii) comprises using an enzyme, e.g. in an enzymatic pretreatment and/or another spheroplasting procedure. In one embodiment, said pretreating in step ii) comprises using an enzyme, e.g. in an enzymatic pretreatment and/or another spheroplasting procedure, which is produced by and/or obtained from a filamentous fungus, such as *Trichoderma sp., Aspergillus sp.,* e.g. *A. niger* and *A. awamori; Penecillium sp.; Aureobasidium sp.; Phaenarocaete sp.; Fusarium sp.; Streptomyces sp.;* or *Bacillus sp.*

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by $\pm 20\%$, $\pm 15\%$, $\pm 10\%$, and for example $\pm 5\%$. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

BRIEF DESCRIPTION OF THE FIGURES

The present invention is now further described by reference to the following figures. All methods mentioned in the figure descriptions below were carried out as described in detail in the examples.

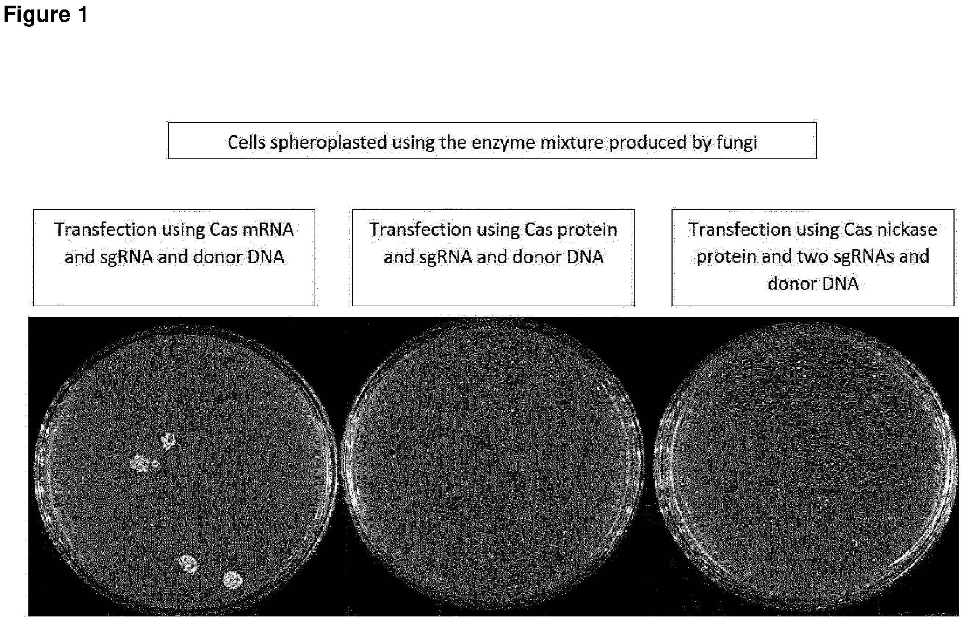

**Figure 1** shows the transfection using mRNA results in more colonies. Positive colonies are labelled with circles. All colonies numbered on plates appeared during the effectiveness period of the selection agent. The unnumbered small colonies are unspecific colonies after the selection agent lost its effect. It is demonstrated that spheroplasting allows for an efficient transfection of GC rich microorganisms, such as oleaginous yeasts e.g. *C. oleaginosus.* Furthermore, it is shown that transfection using a RNA-guided endonuclease in the form of mRNA is even more efficient than transfected and using the RNA-guided endonuclease in the form of protein. It is also shown that pretreating a GC rich microorganism, e.g. an oleaginous microorganism, using spheroplasting, in combination with transacting the organism using a RNA-guided endonuclease in the form of mRNA, synergistically increase the efficiency of genetically modifying the GC rich microorganism.

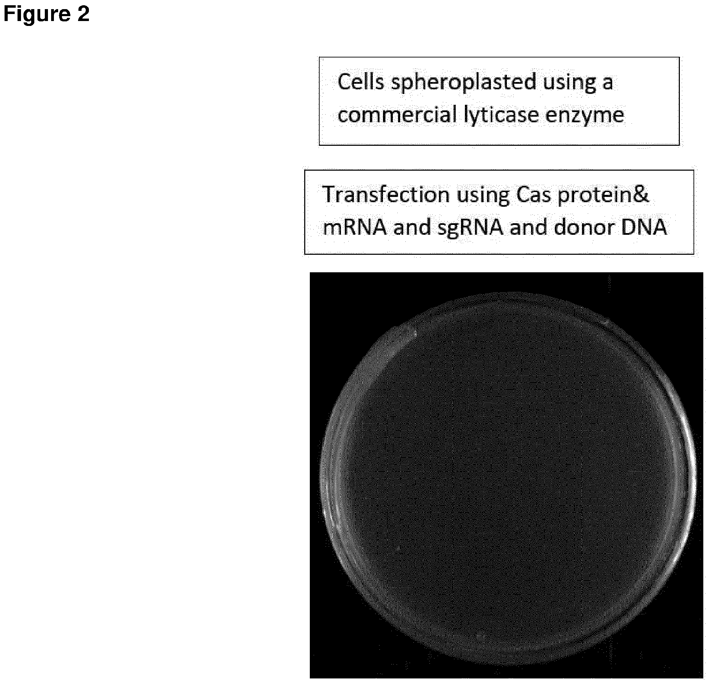

**Figure 2** shows that no transformants are obtained when spheroplasting using a commercially lyticase enzyme. Thus, spheroplasting GC rich microorganisms such as oleaginous yeasts using enzyme mixes adapted to the respective microorganism is surprisingly superior to spheroplasting with commercially available enzymes. Unexpectedly, the spheroplasting procedure used in a method of the invention allows for highly efficient transfection of GC rich microorganisms, such as oleaginous yeasts e.g. *C. oleaginosus,* using a RNA-guided endonuclease.

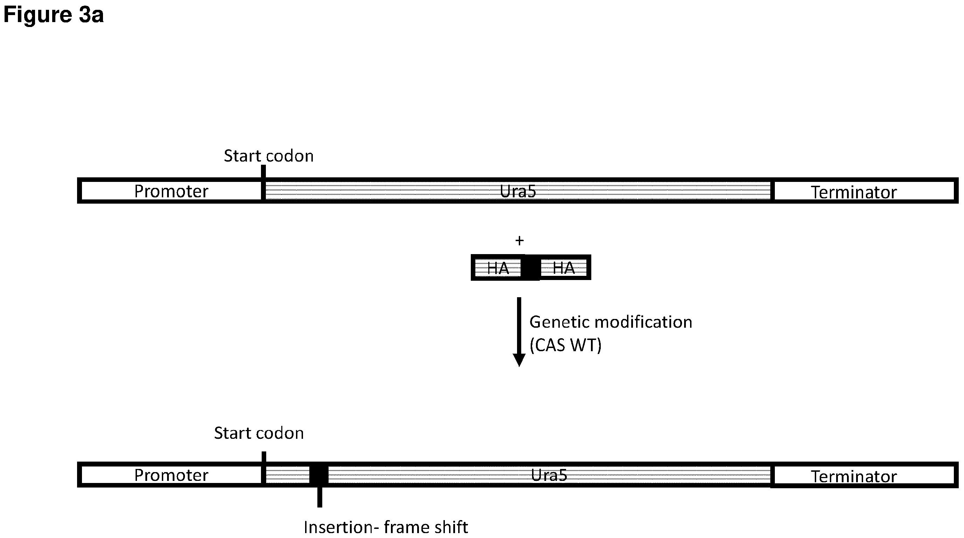

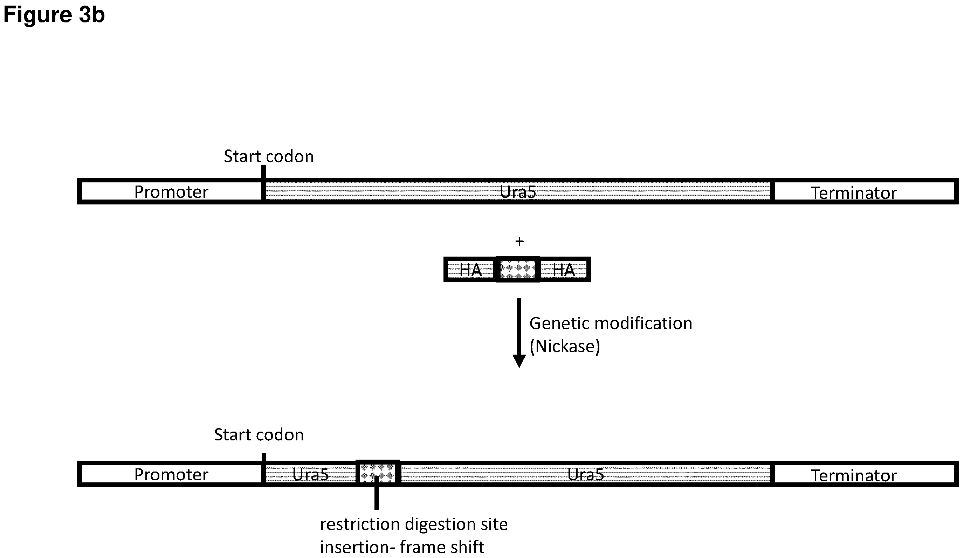

**Figure 3** shows a modification on an exemplary target site on the Ura5 loci in the genomic DNA. A) Genetic modification using Cas wildtype. In this example, the ura5 gene is knocked out by creating a targeted frame shift using CAS9. This method allows for targeted gene knockout at the selected loci. B) Genetic modification using Cas nickase. In this example, the ura5 gene is knocked out by creating a targeted frame shift using CAS Nickase. This method allows for targeted gene knockout at the selected loci.

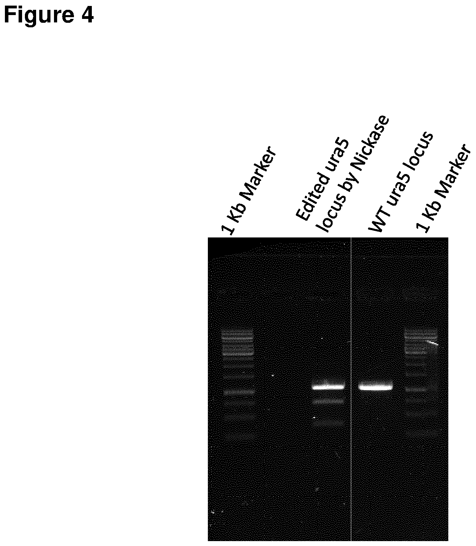

**Figure 4** shows the DNA gel electrophoresis of Ura5 gene from the wild type and the mutant (engineered by nickase) after restriction digestion. Agarose gel electrophoresis: wild type ura5 locus and edited ura5 locus, both treated by restriction digestion enzyme (a non cutter enzyme in WT ura5 gene). The Donor DNA for the knockout of ura5 by the nickases in this example contained a non-cutter restriction digestion site. The ura5 gene of the mutant and wild type were amplified and digested by the respective enzyme.

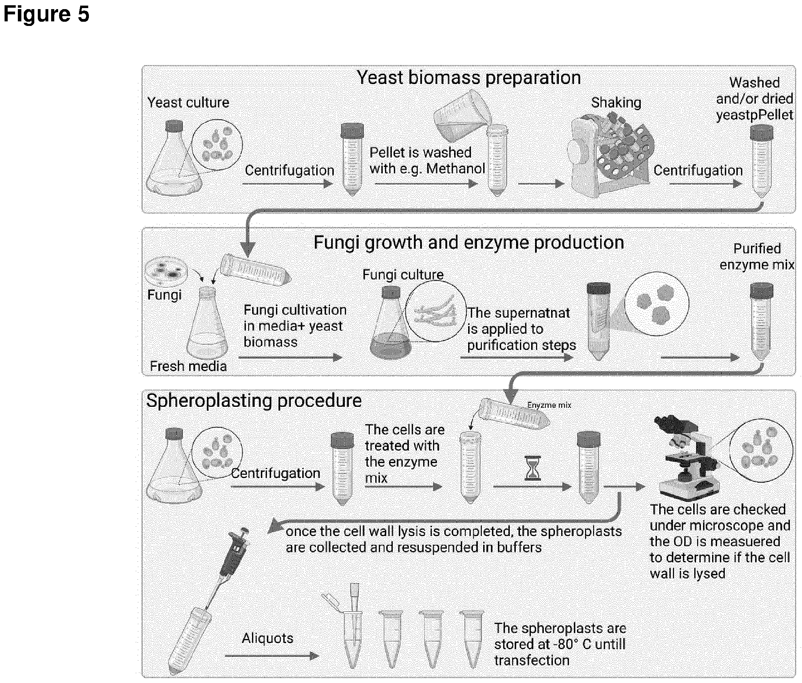

**Figure 5** shows an exemplary spheroplasting procedure used in a method of the invention. The spheroplasting procedure allows for pretreating the microorganism such that the microorganisms can be efficiently transfected.

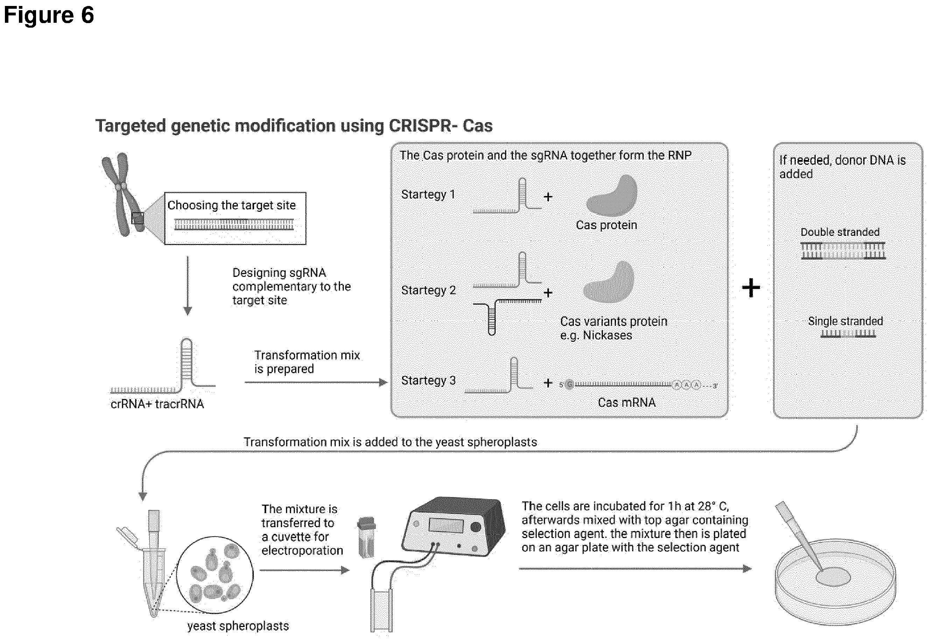

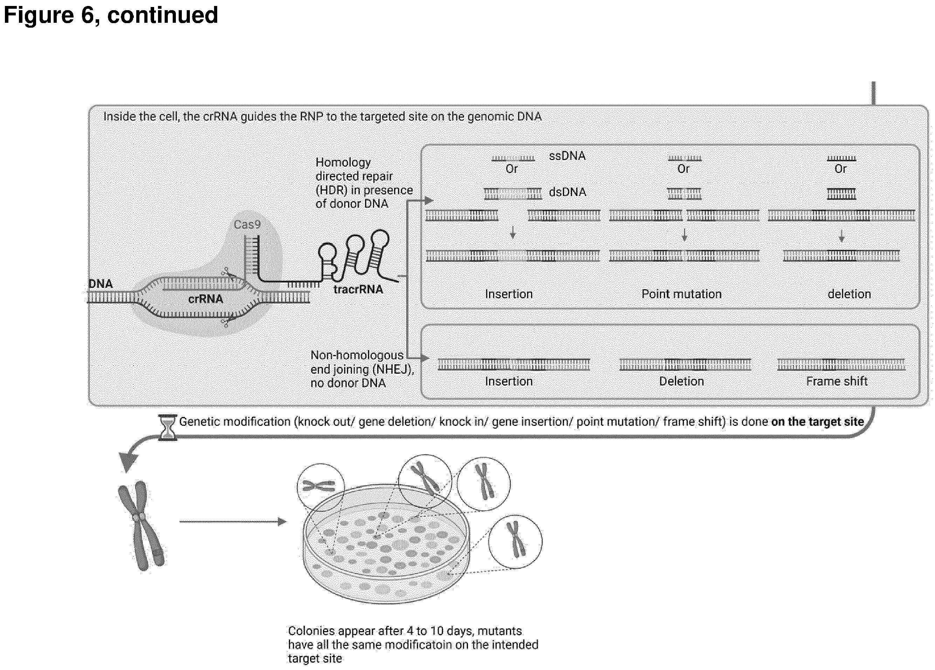

**Figure 6** shows a schematic representation of targeted genetic modification of a GC rich microorganism using CRISPR-Cas. The method of the invention comprising spheroplasting allows to efficiently transfect GC rich microorganisms, such as oleaginous microorganisms having a high GC content.

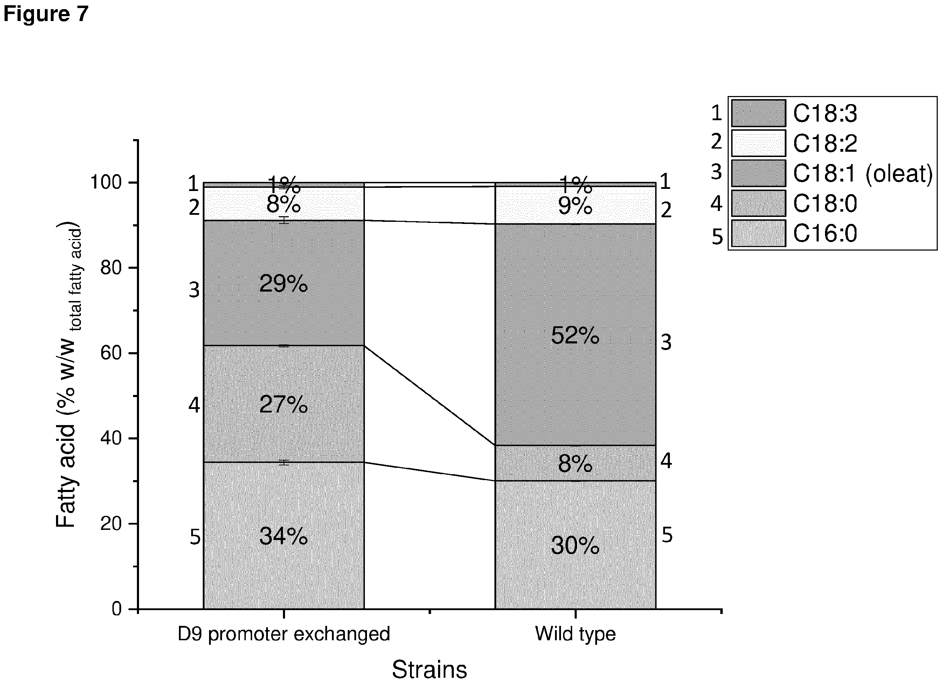

**Figure 7** shows the fatty acid profile of the engineered strain via CRISPR method and the wild type in minimal nitrogen media. The replacement of the promoter of delta-9 desaturase with the TEF promoter resulted in lower C18:1 and higher C18:0. Thus, the method of the invention allows to effectively modify the fatty acid profile of a GC rich microorganism and to prepare an oil having a specific fatty acid profile.

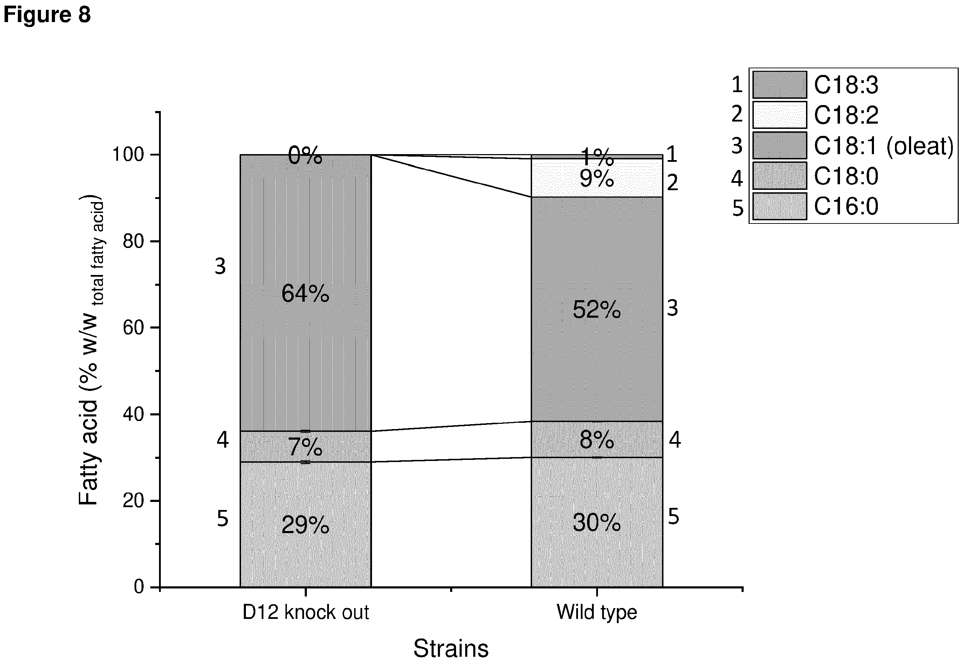

**Figure 8** shows that the knockout of Delta-12 desaturase gene in a GC rich microorganism, particularly an oleaginous microorganism, resulted in absence of C18:2 and C18:3 in the final fatty acid profile of this mutant. Accordingly, the method of the invention allows to effectively modify the fatty acid profile of a GC rich microorganism and allows to prepare an oil having a specific fatty acid profile.

In the following, reference is made to the examples, which are given to illustrate, not to limit the present invention.

EXAMPLES

**Example 1:** *Transfection of an oleaginous microorganism using a RNA-guided endonuclease.*

Transfection was performed as shown in Figure 6.

The spheroplasts were mixed with endonuclease mRNA or protein along with the Donor DNA and sgRNA, electroporated and plated on selection plates within a top agar layer harboring the selection agent. The plates were incubated between 28 and 30 degrees until colonies were obtained.

The inventors have demonstrated that transfection using Cas mRNA is more efficient than transfection using Cas protein. The inventors have further demonstrated that transfection using two sgRNAs is more efficient than transfection using only one type of sgRNA.

**Example 2:** *Spheroplasting increases transfection efficiency.*

Spheroplasting was performed as shown in Figure 5.

Spheroplasts prepared using the commercial enzyme and the tailored enzyme mix. Both spheroplasts were mixed with endonuclease mRNA or protein along with the Donor DNA and sgRNA, electroporated and plated on selection plates within a top agar layer harboring the selection agent. The plates were incubated between 28 and 30 degrees until colonies were obtained.

The inventors have demonstrated that pretreating a GC rich microorganism, particularly an oleaginous microorganism, e.g. by spheroplasting, increases the transfection efficiency. The microorganisms treated with the commercial enzyme did not result in any transformants. Particularly, spheroplasting using a tailored enzyme mix, such as an enzyme mix produced by a fungus, allows a much higher transfection efficiency than a transfection without prior spheroplasting or a spheroplasting using a commercial lyticase enzyme.

**Example 3:** *The method of the invention successfully integrates donor DNA in the genomic DNA of an oleaginous microorganism.*

The spheroplasts were mixed with endonuclease mRNA or protein along with the Donor DNA and sgRNA, electroporated and plated on selection plates within a top agar layer harboring the selection agent. The plates were incubated between 28 and 30 degrees until colonies were obtained.

As shown in Figure 3, an exemplary target site was genetically modified. Sequencing confirmed a successful modification of an exemplary target site on the Ura5 locus in the genomic DNA of *Cutaneotrichosporon oleaginosus.*

REFERENCES

[1] Gorner, C. et al. Genetic engineering and production of modified fatty acids by the nonconventional oleaginous yeast Trichosporon oleaginosus ATCC 20509. Green Chemistry 18, 2037-2046, doi:10.1039/C5GC01767J (2016).

[2] Bracharz, F., Beukhout, T., Mehlmer, N. & Brück, T. Opportunities and challenges in the development of Cutaneotrichosporon oleaginosus ATCC 20509 as a new cell factory for custom tailored microbial oils. Microbial cell factories 16, 178-178, doi:10.1186/s12934-017-0791-9 (2017).

The features of the present invention disclosed in the specification, the claims, and/or in the accompanying figures may, both separately and in any combination thereof, be material for realizing the invention in various forms thereof.

**Claims**

1. A method of genetically modifying a GC rich microorganism, optionally for increasing the production of acetyl-CoA-based hydrophobic compounds in said microorganism, comprising the following steps:

  i) providing a GC rich microorganism, preferably an oleaginous microorganism, more preferably an oleaginous yeast;
  ii) optionally, pretreating said GC rich microorganism; wherein, preferably, said pretreating comprises spheroplasting said GC rich microorganism;
  iii) transforming said GC rich microorganism with a RNA-guided endonuclease, at least one guide RNA, and optionally a donor DNA;
  iv) optionally, selecting a transformed cell of said GC rich microorganism;
  v) obtaining a genetically modified GC rich microorganism.

2. The method according to claim 1, wherein said GC rich microorganism is selected from yeasts, fungi, bacteria and microalgae; wherein, preferably, said GC rich microorganism is an oleaginous microorganism; wherein, more preferably, said GC rich microorganism is an oleaginous yeast; wherein, even more preferably, said GC rich microorganism is selected from *Rhodosporidium sp., Yarrowia sp., Rhodotorula sp., Candida sp., Lipomyces sp., Cutaneotrichosporon sp., Trichosporon sp.,* preferably selected from *Cutaneotrichosporon sp.,* more preferably *Cutaneotrichosporon oleaginosus* (ATCC 20509); and/or

wherein said GC rich microorganism has a guanine-cytosine (GC) content of at least 50%, preferably at least 55%, more preferably at least 58%, even more preferably at least 60%.

3. The method according to claim 1 or 2, wherein said RNA-guided endonuclease is a CRISPR-associated (Cas) endonuclease, preferably selected from Cas9, Cas1, Cas2, Cas4, Cas3, Cas10, Casi2, Cas13, Csm, scfi, or variants thereof, e.g. Cas12a, dCas9, D10A CAS9 nickase or H840A CAS9 nickase.

4. The method according to any one of the foregoing claims, wherein said transforming in step iii) comprises applying said RNA-guided endonuclease in the form of a RNA-guided endonuclease protein, in the form of a DNA encoding said RNA-guided endonuclease, or in the form of a mRNA encoding said RNA-guided endonuclease to said GC rich microorganism, preferably in the form of a mRNA encoding said RNA-guided endonuclease.

5. The method according to any one of the foregoing claims, wherein said at least one guide RNA comprises CRISPR RNA (crRNA), trans-activating CRISPR RNA (tracrRNA), and/or single-guide RNA (sgRNA); wherein, preferably, said at least one guide RNA comprises a first guide RNA and a second guide RNA, wherein a sequence of said first guide RNA is different from a sequence of said second guide RNA.

6. The method according to any one of the foregoing claims, wherein said donor DNA comprises a DNA repair template, a DNA encoding a selection marker, and/or a gene or sequence of interest, e.g. a gene involved in the production of a target compound such as a fatty acid.

7. The method according to any one of the foregoing claims, wherein said transforming in step iii) comprises applying said RNA-guided endonuclease and said at least one guide RNA, preferably jointly, in the form of a ribonucleoprotein complex.

8. The method according to any one of the foregoing claims, wherein said transforming in step iii) comprises applying said RNA-guided endonuclease in the form of a mRNA encoding said RNA-guided endonuclease, and wherein said at least one guide RNA comprises sgRNA.

9. The method according to any one of the foregoing claims, wherein said pretreating in step ii) comprises an enzymatic pretreatment, chemical pretreatment, and/or another spheroplasting procedure; wherein, preferably, said pretreating in step ii) comprises an enzymatic pretreatment performed using at least one enzyme selected from glycosyl hydrolases, cellulase, hemicellulase, mannanase, xyloglucanse, xylanase, glucanase, glucosidase, arabinase, amylase, fructanase, laminarase, hydrolase, protease, or any combination thereof, preferably a combination of cellulase, hemicellulase, mannanase, xyloglucanse, xylanase, glucanase, and glucosidase.

10. The method according to any one of the foregoing claims, wherein said pretreating in step ii) comprises a treatment of said microorganism with a hydrolase alone, or a hydrolase in combination with/followed by a protease;

wherein, optionally, said hydrolase is selected from hydrolases produced by a fungus, preferably a filamentous fungus, more preferably a fungus selected from the *Trichoderma sp., Aspergillus sp., Penicillium sp., Aureobasillium sp.* and *Fusarium sp.,* even more preferably *Trichoderma reesei;* and/or

wherein, optionally, said protease is selected from proteases produced by *Aspergillus sp., Streptomyces sp.* or *Bacillus sp.*

11. The method according to any one of the foregoing claims, wherein said transforming in step iii) is performed using electroporation; PEG-based or other nanoscale carrier-based transformation; biological ballistics; glass bead transformation; vesicle-mediated delivery; a viral transfection system, e.g. lentivirus (LVs), adenovirus (AdV), or adeno-associated virus (AAV); liposomal delivery, e.g. lipofection; a chemical transfection technique; or combinations thereof;

preferably using electroporation and/or PEG-based transformation; more preferably using electroporation.

12. The method according to any one of the foregoing claims, wherein said method comprises said selecting in step iv), wherein said selecting in step iv) comprises subjecting said microorganism to a selective agent selective for a selection marker, optionally a selection marker encoded by said donor DNA;

wherein, preferably, said selecting comprises subjecting said microorganism to a selective agent at a concentration in the range of from 10 % to 90 %, preferably 10 % to 70 %, more preferably 10 % to 60 %, of the minimum selectable concentration; and/or subjecting said microorganism to a selective agent at a concentration in the range of from 90 % to 100 %, preferably 99 % to 100 %, of the minimum selectable concentration;

wherein, more preferably, said selecting comprises subjecting said microorganism to said selective agent at a concentration in the range of from 10 % to 90 %, preferably 10 % to 70 %, more preferably 10 % to 60 %, of the minimum selectable concentration in a first medium, e.g. a liquid medium or solid medium, preferably in an upper layer of agar, and, optionally subsequently, subjecting said microorganism to a selective agent at a concentration in the range of from 90 % to 100 %, preferably 99 % to 100 % in a second medium, e.g. a liquid medium or solid medium, preferably in a lower layer of agar.

13. A genetically modified GC rich microorganism, preferably an oleaginous microorganism, more preferably an oleaginous yeast, even more preferably *Cutaneotrichosporon oleaginosus,* comprising a RNA-guided endonuclease, at least one guide RNA, and optionally a donor DNA; wherein, optionally, said RNA-guided endonuclease and said at least one guide RNA are present in said cell in the form of a ribonucleoprotein complex.

14. A composition comprising

- a RNA-guided endonuclease; optionally a RNA-guided endonuclease protein, a DNA encoding said RNA-guided endonuclease, or a mRNA encoding the RNA-guided endonuclease, preferably a mRNA encoding the RNA-guided endonuclease;
wherein, preferably, said RNA-guided endonuclease is a CRISPR-associated (Cas) endonuclease, more preferably selected from Cas9, Cas1, Cas2, Cas4, Cas3, Cas10, Casi2, Cas13, Csm, scfi, or variants thereof, e.g. Cas12a, dCas9, D10A CAS9 nickase or H840A CAS9 nickase, even more preferably Cas9 endonuclease such as Cas9 endonuclease having a sequence of any of SEQ ID NO. 1-4;
- at least one guide RNA (gRNA); preferably crRNA, tracrRNA, and/or sgRNA;
wherein, optionally, said gRNA comprises a tracrRNA sequence of SEQ ID NO. 5 and/or comprises a crRNA sequence of any one of SEQ ID NOs. 14-26;
- optionally, donor DNA; preferably comprising a DNA repair template, a DNA encoding a selection marker, and/or a gene or sequence of interest, e.g. a gene involved in the production of a target compound such as a fatty acid;

wherein, optionally, said selection marker is Ura5;
wherein, optionally, said gene or sequence of interest encodes delta-9-desaturase, delta-12-desaturase, elongase, oleat hydratase, aldo keto reductase promoter, aldo keto reductase terminator, transcription elongation factor 2 promoter, TEF promoter, and/or TEF terminator;
wherein, optionally, said donor DNA comprises a sequence of any of SEQ ID NO. 6-13 and 27.

15. A plasmid or plasmid collection comprising

- a mRNA encoding a RNA-guided endonuclease;
wherein, preferably, said RNA-guided endonuclease is Cas9 endonuclease, more preferably Cas9 endonuclease having a sequence of any of SEQ ID NO. NO. 1-4;
- at least one guide RNA (gRNA); preferably crRNA, tracrRNA, and/or sgRNA;
wherein, optionally, said gRNA comprises a tracrRNA sequence of SEQ ID NO. 5 and/or comprises a crRNA sequence of any one of SEQ ID NOs. 14-26;
- optionally, donor DNA, e.g. a DNA repair template;
wherein, optionally, said donor DNA has a sequence of any of SEQ ID NO. 6-13 and 27.

16. A method of preparing a target compound, e.g. acetyl-CoA or an acetyl-CoA-based hydrophobic compound, and/or an oil having a specific fatty acid profile, e.g. high oleic oil, using a genetically modified GC rich microorganism, preferably oleaginous microorganism, more preferably oleaginous yeast, wherein said method comprises:

a) providing a genetically modified GC rich microorganism, preferably oleaginous microorganism, more preferably oleaginous yeast, using a method of any one of claims 1-12; wherein said method comprises transforming said microorganism with a donor DNA, wherein said donor DNA comprises a gene or sequence of interest, e.g. a gene involved in the production of said target compound and/or specific fatty acid;
b) growing said genetically modified GC rich microorganism;
c) obtaining said target compound and/or oil having a specific fatty acid profile; wherein, preferably, said target compound is selected from saturated short-chain fatty acids, saturated medium-chain fatty acids, saturated long-chain fatty acids, monounsaturated fatty acids, polyunsaturated fatty acids, functionalized fatty acids, ergosterol, ergosterol derivatives, terpenes, alkaloids, acetyl-CoA-based synthetic compounds, tocochromanols, e.g. $\alpha$-tocopherol and $\alpha$-tocotrienol, monoterpenoids, sesquiterpenoids, diterpenoids, squalene, carotenoids, triterpenes, pheophytins, vitamins, citric acid, volatile fatty acids, oxalic acid, lactic acid, malic acid, and exopolysaccharides.

## Figure 1

| Cells spheroplasted using the enzyme mixture produced by fungi |
|---|

| Transfection using Cas mRNA and sgRNA and donor DNA | Transfection using Cas protein and sgRNA and donor DNA | Transfection using Cas nickase protein and two sgRNAs and donor DNA |
|---|---|---|

**Figure 2**

Cells spheroplasted using a commercial lyticase enzyme

Transfection using Cas protein& mRNA and sgRNA and donor DNA

EP 4 202 043 A1

**Figure 3a**

# Figure 3b

Start codon

| Promoter | Ura5 | Terminator |

+

| HA | | HA |

Genetic modification
(Nickase)

Start codon

| Promoter | Ura5 | | Ura5 | Terminator |

restriction digestion site
insertion- frame shift

Figure 4

# Figure 5

# Figure 6

**Targeted genetic modification using CRISPR- Cas**

Choosing the target site

Designing sgRNA complementary to the target site

Transformation mix is prepared

crRNA+ tracrRNA

The Cas protein and the sgRNA together form the RNP

Startegy 1 + Cas protein

Startegy 2 + Cas variants protein e.g. Nickases

Startegy 3 + Cas mRNA

If needed, donor DNA is added

Double stranded

Single stranded

Transformation mix is added to the yeast spheroplasts

The mixture is transferred to a cuvette for electroporation

yeast spheroplasts

The cells are incubated for 1h at 28° C, afterwards mixed with top agar containing selection agent. the mixture then is plated on an agar plate with the selection agent

# Figure 6, continued

Inside the cell, the crRNA guides the RNP to the targeted site on the genomic DNA

Homology directed repair (HDR) in presence of donor DNA

ssDNA
Or
Or
Or

dsDNA

Insertion

Point mutation

deletion

Non-homologous end joining (NHEJ), no donor DNA

Insertion

Deletion

Frame shift

Cas9

DNA

crRNA

tracrRNA

Genetic modification (knock out/ gene deletion/ knock in/ gene insertion/ point mutation/ frame shift) is done **on the target site**

Colonies appear after 4 to 10 days, mutants have all the same modificatoin on the intended target site

Figure 7

EP 4 202 043 A1

**Figure 8**

Europäisches
Patentamt

European
Patent Office

Office européen
des brevets

# EUROPEAN SEARCH REPORT

**Application Number**

EP 21 21 7144

## DOCUMENTS CONSIDERED TO BE RELEVANT

| Category | Citation of document with indication, where appropriate, of relevant passages | Relevant to claim |
|---|---|---|
| X | JIAO XIANG ET AL: "Developing a CRISPR/Cas9 System for Genome Editing in the Basidiomycetous Yeast Rhodosporidium toruloides", BIOTECHNOLOGY JOURNAL, [Online] vol. 14, no. 7, 3 June 2019 (2019-06-03), page 1900036, XP055928876, DE ISSN: 1860-6768, DOI: 10.1002/biot.201900036 Retrieved from the Internet: URL:https://onlinelibrary.wiley.com/doi/full-xml/10.1002/biot.201900036> [retrieved on 2022-06-08] * abstract; introduction, col. 1-col. 2 bridging sentence; experimental section, 2.6, 3.2 and 3.4; Fig. 3A; page 6, last par. * | 1-16 |

-----

-/--

**CLASSIFICATION OF THE APPLICATION (IPC)**

INV.
C12N9/22
C12P7/64
C12N15/52
C12N15/90
C12N15/11

**TECHNICAL FIELDS SEARCHED (IPC)**

C12R
C12N
C12P

The present search report has been drawn up for all claims

| Place of search | Date of completion of the search | Examiner |
|---|---|---|
| Munich | 9 June 2022 | Dumont, Elisabeth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 21 21 7144

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WEN ZHIQIANG ET AL: "Rhodosporidium toruloides - A potential red yeast chassis for lipids and beyond", FEMS YEAST RESEARCH, vol. 20, no. 5, 1 August 2020 (2020-08-01) , page 38, XP55928880, GB, NL ISSN: 1567-1356, DOI: 10.1093/femsyr/foaa038 Retrieved from the Internet: URL:https://watermark.silverchair.com/foaa 038.pdf?token=AQECAHi208BE49Ooan9kkhW_Ercy 7Dm3ZL_9Cf3qfKAc485ysgAAAuAwggLcBgkqhkiG9w 0BBwagggLNMIICyQIBADCCAsIGCSqGSIb3DQEHATAe BglghkgBZQMEAS4wEQQMmANQ3u4Ke0khO34FAgEQgI ICk7WhyTK0fH5-w6GhqjiAARCk57sczznN3IrBkCcd 9y42OJayfrB-TcHQD3-dtTNKZ6gOj2CW6ZhUmKMdcX wLoFf8rSOp> * abstract; page 2 col. 1 par. 1 and col. 2 par. 3 * ----- | 1-16 | |
| X | YIP BON ET AL: "Recent Advances in CRISPR/Cas9 Delivery Strategies", BIOMOLECULES, vol. 10, no. 6, 30 May 2020 (2020-05-30), page 839, XP55928901, DOI: 10.3390/biom10060839 Retrieved from the Internet: URL:https://www.ncbi.nlm.nih.gov/pmc/artic les/PMC7356196/pdf/biomolecules-10-00839.p df> * the whole document * * in particular abstract; Fig. 1A; page 8, 3.3. * ----- -/-- | 14,15 | TECHNICAL FIELDS SEARCHED (IPC) |

The present search report has been drawn up for all claims

| Place of search | Date of completion of the search | Examiner |
|---|---|---|
| Munich | 9 June 2022 | Dumont, Elisabeth |

EPO FORM 1503 03.82 (P04C01)

page 2 of 4

Europäisches
Patentamt

European
Patent Office

Office européen
des brevets

**EUROPEAN SEARCH REPORT**

Application Number

EP 21 21 7144

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KAWAI SHIGEYUKI ET AL: "Transformation of Saccharomyces cerevisiae and other fungi : Methods and possible underlying mechanism", BIOENGINEERED BUGS, vol. 1, no. 6, 1 November 2010 (2010-11-01), pages 395-403, XP55928951, US ISSN: 1949-1018, DOI: 10.4161/bbug.1.6.13257 Retrieved from the Internet: URL:https://www.ncbi.nlm.nih.gov/pmc/articles/PMC3056089/pdf/bbug0106_0395.pdf> * the whole document, in particular abstract; page 395, last par.-page 396, col. 2 par. 1; Table 1; pages 401-402, Conclusion * | 1-16 | |
| Y,D | GÖRNER CHRISTIAN ET AL: "Genetic engineering and production of modified fatty acids by the nonconventional oleaginous yeast Trichosporon oleaginosus ATCC 20509", GREEN CHEMISTRY, vol. 18, no. 7, 1 January 2016 (2016-01-01), pages 2037-2046, XP55928473, GB ISSN: 1463-9262, DOI: 10.1039/C5GC01767J Retrieved from the Internet: URL:https://pubs.rsc.org/en/content/articlepdf/2016/gc/c5gc01767j> * abstract; page 2038, col. 2, second full par.; page 2039, col. 1, par.2 and col. 2 par. 2; Fig. 2; page 2045, col. 1 last par. * | 1-16 | TECHNICAL FIELDS SEARCHED (IPC) |

-/--

The present search report has been drawn up for all claims

| Place of search | Date of completion of the search | Examiner |
|---|---|---|
| Munich | 9 June 2022 | Dumont, Elisabeth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

**EUROPEAN SEARCH REPORT**

Application Number

EP 21 21 7144

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | FELIX BRACHARZ ET AL: "Opportunities and challenges in the development of Cutaneotrichosporon oleaginosus ATCC 20509 as a new cell factory for custom tailored microbial oils", MICROBIAL CELL FACTORIES, vol. 16, no. 1, 25 October 2017 (2017-10-25), XP055465750, DOI: 10.1186/s12934-017-0791-9 * abstract; page 11, col. 1 last par.-page 12 col. 1 par.1 * | 1-16 | |
| Y | CHATTOPADHYAY ATRAYEE ET AL: "Recent advances in lipid metabolic engineering of oleaginous yeasts", BIOTECHNOLOGY ADVANCES, ELSEVIER PUBLISHING, BARKING, GB, vol. 53, 23 February 2021 (2021-02-23), XP086877470, ISSN: 0734-9750, DOI: 10.1016/J.BIOTECHADV.2021.107722 [retrieved on 2021-02-23] * abstract; page 2, par. 2.5.; page 12, par. 6.2. * | 1-16 | |
| A | KOIVURANTA KARI ET AL: "Enhanced Triacylglycerol Production With Genetically Modified Trichosporon oleaginosus", FRONTIERS IN MICROBIOLOGY, vol. 9, 1 January 2018 (2018-01-01), page 1337, XP55928832, DOI: 10.3389/fmicb.2018.01337 Retrieved from the Internet: URL:https://www.ncbi.nlm.nih.gov/pmc/articles/PMC6021488/pdf/fmicb-09-01337.pdf> * abstract * | 1-16 | TECHNICAL FIELDS SEARCHED (IPC) |

The present search report has been drawn up for all claims

| Place of search | Date of completion of the search | Examiner |
|---|---|---|
| Munich | 9 June 2022 | Dumont, Elisabeth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GORNER, C. et al.** Genetic engineering and production of modified fatty acids by the nonconventional oleaginous yeast Trichosporon oleaginosus ATCC 20509. *Green Chemistry,* 2016, vol. 18, 2037-2046

- **BRACHARZ, F. ; BEUKHOUT, T. ; MEHLMER, N. ; BRÜCK, T.** Opportunities and challenges in the development of Cutaneotrichosporon oleaginosus ATCC 20509 as a new cell factory for custom tailored microbial oils. *Microbial cell factories,* 2017, vol. 16, 178-178